# EUROPÄISCHE PATENTSCHRIFT

(11) **EP 3 537 987 B1**
(45) Veröffentlichungstag und Bekanntmachung des Hinweises auf die Patenterteilung: **16.09.2020**
(21) Anmeldenummer: 17800489.1
(22) Anmeldetag: 13.11.2017
(51) Int. Cl.: A61B 17/00, A61B 17/3207

(54) **VORRICHTUNG ZUR ENTFERNUNG VON ORGANEN AUS DEM MENSCHLICHEN ODER TIERISCHEN KÖRPER**
DEVICE FOR REMOVAL OF ORGANS FROM THE HUMAN OR ANIMAL BODY
DISPOSITIF POUR ENLEVER DES ORGANES DU CORPS HUMAIN OU ANIMAL

(30) Priorität: 11.11.2016 DE 202016006899 U
(43) Veröffentlichungstag der Anmeldung: 18.09.2019
(73) Patentinhaber: Büscherhoff, Bernd, 49439 Steinfeld (DE); Holthaus, Bernd, 49448 Quernheim (DE)
(72) Erfinder: BÜSCHERHOFF, Bernd, 49439 Steinfeld (DE)
(74) Vertreter: Eisenführ Speiser
(86) Internationale Anmeldenummer: PCT/EP2017/079067
(87) Internationale Veröffentlichungsnummer: WO 2018/087368

(56) Entgegenhaltungen:
- WO-A1-2015/118542
- DE-A1- 10 229 137
- DE-A1-102010 024 360
- US-A- 5 215 521
- US-A- 5 836 953
- US-A1- 2004 242 960

## Beschreibung

Die Erfindung betrifft eine Vorrichtung zur Entfernung von Organen aus dem menschlichen oder tierischen Körper.

Eine solche Vorrichtung dient zur Entsorgung von inneren Organen, die insbesondere aufgrund von Krankheiten oder sonstigen negativen Einflüssen in Mitleidenschaft gezogen worden sind und ihre Funktion nicht mehr ordnungsgemäß ausführen können oder gar abgestorben sind. Insbesondere ist eine solche Vorrichtung dazu bestimmt, einen krankhaften Uterus zu entfernen. Zwar arbeiten viele Vorrichtungen der eingangs genannten Art endoskopisch und umfassen mindestens zwei Endoskope, von denen ein Endoskop eine Zange enthält, um das zu entfernende Organ abzutrennen. Jedoch war es mit den bisher bekannten Vorrichtungen nicht oder nur mit sehr hohem Aufwand möglich, innerhalb des Körpers im Bereich des zu entfernenden Organs eine sterile Umgebung zu schaffen, und verblieben Reste und Restpartikel des entfernten Organs im Körper, was zu einer unerwünschten Kontamination innerhalb des Körpers führte. Insbesondere besteht das Problem bei den bekannten Vorrichtungen darin, dass das abgeschnittene Organ aufgrund der Schwerkraft in den darunter befindlichen Abschnitt des Körpers fällt, was nicht nur die Kontamination begünstigt, sondern auch zu Problemen bei der Sterilität führt.

Die US 5,836,953 offenbart eine Vorrichtung zur Entfernung von Organen aus dem menschlichen oder tierischen Körper, mit einem Rohr, das ein proximales Ende und ein distales Ende aufweist und zum teilweisen Einführen mit seinem proximalen Ende in den Körper vorgesehen ist, wobei das distale Ende des Rohres an eine Saugluftquelle anschließbar und das proximale Ende des Rohres für einen Saugeingriff mit dem zu entfernenden Organ ausgebildet ist, mit einer das Rohr zumindest abschnittsweise umschließenden Schlauchhülle, die ein proximales Ende und ein distales Ende, mit dem sie am Rohr befestigt ist, aufweist, mit einer Handhabungseinrichtung, die am Rohr angeordnet und ausgebildet ist, die Schlauchhülle an ihrem proximalen Ende zu öffnen oder aufzuweiten und um das Organ herum zu führen oder zu legen, mit einer Schließeinrichtung, die zum Verschließen des proximalen Endes der Schlauchhülle ausgebildet ist, und mit einer Zerkleinerungseinrichtung, die innerhalb des Rohres, vorzugsweise im Bereich seines proximalen Endes, vorgesehen ist.

Die DE 102 29 137 A1 offenbart ein chirurgisches Instrument, das insbesondere zum Behandeln von Zysten geeignet ist und einen Saugkopf besitzt, der mindestens eine Saugöffnung aufweist und zum dichten Anliegen an ein Körpergewebe eingerichtet ist. Die Säugöffnung kann über eine Unterdruckleitung mit einer Unterdruckquelle in Verbindung gebracht werden.

Aus der DE 10 2010 024 360 A1 ist ein Uretersteinsauginstrument mit einem Schaft bekannt, der von einem an eine Saugeinrichtung anschließbaren Saugkanal durchlaufen ist, wobei distal vom Schaft ein Fangbehälter mit einer distalen und einer proximalen Öffnung angeordnet ist, von denen die proximale Öffnung an das distale Ende des Saugkanales angeschlossen ist.

Es ist eine Aufgabe der vorliegenden Erfindung, eine verbesserte Vorrichtung der eingangs genannten Art vorzuschlagen, mit der sich problemlos innerhalb des Körpers im Bereich des zu entfernenden Organs zumindest im Wesentlichen eine sterile Umgebung schaffen und des Weiteren eine Kontamination mit Resten und Restpartikeln des entfernten Organs zumindest im Wesentlichen vermeiden lässt und mit der sich insbesondere das Erfassen und Halten des zu entfernenden Organs und dessen Zerkleinerung vereinfachen lässt.

Die Aufgabe wird erfindungsgemäß gelöst mit einer Vorrichtung zur Entfernung von Organen aus dem menschlichen oder tierischen Körper, mit einem Rohr, das ein proximales Ende und ein distales Ende aufweist und zum teilweisen Einführen mit seinem proximalen Ende in den Körper vorgesehen ist, wobei das distale Ende des Rohres an eine Saugluftquelle anschließbar und das proximale Ende des Rohres für einen Saugeingriff mit dem zu entfernenden Organ ausgebildet ist, mit einer das Rohr zumindest abschnittsweise umschließenden Schlauchhülle, die ein proximales Ende und ein distales Ende, mit dem sie am Rohr befestigt ist, aufweist, mit einer Handhabungseinrichtung, die bevorzugt am Rohr angeordnet und ausgebildet ist, die Schlauchhülle an ihrem proximalen Ende zu öffnen oder aufzuweiten und um das Organ herum zu führen oder zu legen, mit einer Schließeinrichtung, die zum Verschließen des proximalen Endes der Schlauchhülle ausgebildet ist, und mit einer Zerkleinerungseinrichtung, die innerhalb des Rohres, vorzugsweise im Bereich seines proximalen Endes, vorgesehen ist.

Durch die erfindungsgemäße Verwendung einer Schlauchhülle, die an ihrem proximalen Ende zunächst geöffnet oder aufgeweitet, dann um das Organ herum geführt oder gelegt und anschließend an ihrem proximalen Ende verschlossen wird, lässt sich das zu entfernende Organ auf unkomplizierte Weise sicher und vollständig einhausen, wodurch zumindest weitestgehend die geforderte Sterilität erzielt und zugleich eine Kontamination von Resten oder Restpartikeln des zu entfernenden Organs vermieden wird. Dies wird erfindungsgemäß mit einer Handhabungseinrichtung, die an einem in den Körper einzuführenden Rohr angeordnet und ausgebildet ist, die Schlauchhülle an ihrem proximalen Ende zu öffnen oder aufzuweiten und um das Organ herum zu führen oder zu legen, sowie mit einer Schließeinrichtung erreicht, die zum Verschließen des proximalen Endes der Schlauchhülle ausgebildet ist. Unter Anordnung am Rohr ist auch eine Anordnung der Handhabungseinrichtung entlang des Rohres und/oder benachbart zum Rohr sowie des Weiteren eine Anordnung ohne Befestigung am Rohr zu verstehen. Nach Verschließen des proximalen Endes der Schlauchhülle durch die Schließeinrichtung erhält die Schlauchhülle die Form eines Beutels, in der nun das zu entfernende Organ aufgenommen ist.

Für die Entfernung des zu entsorgenden Organs aus dem Körper bietet die Erfindung ebenfalls eine einfache und zugleich wirkungsvolle Lösung, die die hohen Anforderungen an die erforderliche Sterilität erfüllt. Hierzu ist erfindungsgemäß das distale Ende des Rohres an eine Saugluftquelle anschließbar und das proximale Ende des Rohres für einen Saugeingriff mit dem zu entfernenden Organ ausgebildet, wodurch das zu entfernende Organ, das ja von der an ihrem proximalen Ende geschlossenen Schlauchhülle nach Art eines Beutels eingefangen und dadurch von der Umgebung bzw. dem übrigen Körper separiert ist, in das proximale Ende des Rohres eingesogen wird. Des Weiteren ist erfindungsgemäß eine Zerkleinerungseinrichtung vorgesehen, die innerhalb des Rohres im Bereich seines proximalen Endes angeordnet und ausgebildet ist, das zu entfernende Organ zu zerkleinern, während es durch die Saugluftquelle in das proximale Ende des Rohres eingesogen wird. Durch die Zerkleinerung wird das Volumen des zu entfernenden Organs und somit auch der nach Art eines Beutels das Organ aufnehmenden, geschlossenen Schlauchhülle reduziert, wodurch sich das zu entfernende Organ leichter durch das Rohr absaugen lässt.

An dieser Stelle sei noch angemerkt, dass die Bezeichnung "proximal", die laut Duden "der Körpermitte zu gelegen" bedeutet, und die Bezeichnung "distal", die laut Duden "weiter von der Körpermitte entfernt liegend" bedeutet, sich im vorliegenden Kontext auf die Stelle des zu entsorgenden Organs innerhalb des Körpers vor dessen Entfernung beziehen.

Bevorzugte Ausführungen und Weiterbildungen der Erfindung sind in den abhängigen Ansprüchen gekennzeichnet.

So ist es zweckmäßig, die Handhabungseinrichtung gegenüber dem Rohr in dessen Längsrichtung zwischen einer ausgefahrenen proximalen Endstellung und einer zurückgezogenen distalen Endstellung bewegbar anzuordnen, wobei in einer bevorzugten Weiterbildung die Handhabungseinrichtung gegenüber dem Rohr zusätzlich in dessen Querrichtung bewegbar angeordnet ist, um in Abhängigkeit von der jeweiligen Lage des zu entfernenden Organs effektiv die Schlauchhülle um das Organ legen zu können.

Eine besonders bevorzugte Konstruktion der Handhabungseinrichtung zeichnet sich dadurch aus, dass sie einander gegenüberliegende Greifarme aufweist, die jeweils ein proximales Ende und ein distales Ende aufweisen und zum lösbaren Greifen der Schlauchhülle, bevorzugt im Bereich ihres proximalen Endes, ausgebildet sind.

Vorzugsweise weisen die Greifarme, bevorzugt im Bereich deren proximalen Endes, Fixiermittel auf, die ausgebildet sind, die Schlauchhülle, bevorzugt mit ihrem proximalen Ende, an den Greifarmen lösbar zu fixieren.

Zweckmäßigerweise sind die Greifarme in Längsrichtung des Rohres orientiert.

Bevorzugt sind die Greifarme winklig, vorzugsweise etwa quer, zur Längserstreckung des Rohres elastisch ausgebildet. Bei einer bevorzugten Weiterbildung weisen in einem entspannten Zustand der Greifarme die proximalen Enden von jeweils einander gegenüberliegenden Greifarmen einen Abstand voneinander auf, der kleiner als der Durchmesser des Rohres ist, und ist ein zwischen den proximalen und distalen Enden befindlicher mittlerer Abschnitt der Greifarme in Bezug auf das Rohr nach außen gekrümmt, sodass zumindest an ihrer in radialer Richtung am weitesten außenliegenden Stelle der Abstand der mittleren Abschnitte von jeweils einander gegenüberliegenden Greifarmen größer als der Abstand zwischen den proximalen Enden dieser Greifarme und bevorzugt auch noch größer als der Durchmesser des Rohres ist. Eine solche Formgebung der Greifarme ist besonders vorteilhaft, um nicht nur die Schlauchhülle um das Organ herumzuführen oder zu legen, sondern auch im Bereich der mittleren Abschnitte der Greifarme das von der dann geschlossenen Schlauchhülle umgebene Organ sicher zu halten. Des Weiteren sorgt die Elastizität im Zusammenhang mit der Formgebung dafür, dass die Greifarme an ihrem proximalen Ende gegen die durch die Elastizität verursachte Kraft zunächst geöffnet werden, um dabei die Schlauchhülle an deren proximalen Ende zu öffnen oder aufzuweiten, und anschließend die dann durch die Elastizität entstandene Vorspannung genutzt wird, nach Umschließung des Organs die Greifarme mit ihrem proximalen Ende wieder in ihre Schließstellung zu verbringen.

Bei einer weiteren bevorzugten Ausführung ist die Außenseite des Rohres mit in dessen Längsrichtung verlaufenden Führungsnuten versehen, in denen die Greifarme in deren Längsrichtung beweglich aufgenommen sind, und die Anordnung so getroffen, dass in der proximalen Endstellung der Handhabungseinrichtung die Greifarme mit einem sich an ihr proximales Ende anschließenden Abschnitt über das proximale Ende des Rohres hinausragen und somit freiliegen. Dies ermöglicht einen besonders großen Bewegungsspielraum für die Greifarme.

Bevorzugt weist die Handhabungseinrichtung Spreizelemente auf, die ausgebildet sind, die Greifarme während der Bewegung der Handhabungseinrichtung in ihre proximale Endstellung zumindest teilweise voneinander weg zu spreizen, um die Schlauchhülle an ihrem proximalen Ende zu öffnen oder aufzuweiten und um das Organ herumzuführen oder zu legen.

Bei einer bevorzugten Weiterbildung dieser Ausführung sind die Spreizelemente am Rohr im Bereich dessen proximalen Endes angeordnet und weisen eine von der Außenseite des Rohres zu dessen proximalen Ende hin ansteigende Führungsfläche auf, an die die Greifarme in berührende Anlage bringbar sind. Somit ist der radiale Abstand der Führungsflächen der Spreizelemente an deren proximalem Ende bzw. am proximalen Ende des Rohres größer als der Radius des Rohres und demnach der radiale Abstand zwischen den Führungsflächen von einander gegenüberliegenden Spreizelementen an deren proximalem Ende bzw. am proximalen Ende des Rohres größer als der Durchmesser des Rohres. Die Spreizelemente gemäß dieser bevorzugten Weiterbildung wirken demnach wie ein Konus zur Aufspreizung der daran anliegenden Greifarme. Im Falle der Ausbildung von in Längsrichtung verlaufenden Führungsnuten an bzw. in der Außenseite des Rohres können die Spreizelemente bevorzugt in diesen Führungsnuten angeordnet sein und deren Führungsfläche vom Boden der Führungsnuten ansteigen.

Bei einer bevorzugten Weiterbildung sind die Spreizelemente in einem Abstand voneinander angeordnet, ist jedem Greifarm ein Spreizelement zugeordnet und sind zum Lösen der Greifarme von den Spreizelementen das Rohr einerseits und/oder die Greifarme andererseits mit einer Relativbewegung zueinander beaufschlagbar, um die Greifarme in eine Position seitlich neben den Spreizelementen zu verbringen. Somit sorgt ein seitliches Verschieben des Rohres gegenüber den Greifarmen oder ein seitliches Verschieben der Greifarme gegenüber dem Rohr oder ein entsprechend gegenseitiges seitliches Verschieben des Rohres und der Greifarme zueinander für ein Abrutschen der Greifarme von den Spreizelementen und dadurch für ein Schließen der Greifarme.

Bevorzugt sind die Greifarme im Bereich ihres distalen Endes miteinander verbunden, wodurch die Handhabung der Greifarme, insbesondere während deren Bewegung in die proximale Endstellung, vereinfacht wird. Bei einer vorteilhaften Weiterbildung ist ein ringförmiges Element vorgesehen, an dem die Greifarme mit ihrem distalen Ende befestigt sind und das bevorzugt als Handhabungselement zu verwenden ist.

Zweckmäßigerweise ist die Schließeinrichtung an der Handhabungseinrichtung vorzusehen. Bei Verwendung von Greifarmen kann bevorzugt die Schließeinrichtung Schließmittel aufweisen, die an den Greifarmen, bevorzugt an deren freien oder proximalen Enden, vorgesehen sind. Ferner kann vorzugsweise die Schließeinrichtung am proximalen Ende der Schlauchhülle vorgesehene Augen oder Ösen und mindestens einen durch die Augen oder Ösen ziehbaren Faden oder Draht aufweisen, wodurch das Schließen des proximalen Endes der Schlauchhülle nach Art eines Nähvorganges erfolgt.

Für eine besonders effektive Zerkleinerung des zu entfernenden Organs sollte vorzugsweise die Zerkleinerungseinrichtung mindestens ein rotierbar gelagertes Schneidmesser aufweisen.

Bevorzugt ist eine Hülse zur Anordnung auf der Oberfläche des Körpers oder zum Einsetzen in die Oberfläche des Körpers vorzusehen, wobei das Rohr sich durch die Hülse erstreckt und gegenüber der Hülse bewegbar ist. Eine derartige Hülse erlaubt eine bessere Fixierung der Vorrichtung auf der Oberfläche des Körpers und eine einfachere und zugleich genaue Orientierung des Rohres, wenn es in den Körper in Richtung auf das zu entfernende Organ eingeführt wird.

Im Falle der Verwendung von Greifarmen können bevorzugt die Greifarme zwischen der Innenseite der Hülse und der Außenseite des Rohres angeordnet sein und sich in berührender Anlage an der Innenseite der Hülse und der Außenseite des Rohres befinden. Eine solche Anordnung begünstigt eine sichere Führung der Greifarme. Für den Fall der Ausbildung eines zwischen den proximalen und distalen Enden befindlichen mittleren Abschnittes der Greifarme mit einer nach außen gekrümmten Form bewirkt diese Anordnung eine Spreizung aufgrund des gekrümmten Verlaufes der Greifarme. In der zurückgezogenen distalen Endstellung der Handhabungseinrichtung, in der sich die Greifarme in ihrer sog. Ausgangsposition befinden, sind die Greifarme zwangsweise von der Hülse in einen im Wesentlichen vollständig gestreckten Zustand verbracht und somit im Wesentlichen vollständig gestreckt, da der Zwischenraum zwischen der Außenseite des Rohres und der Innenseite der Hülse keinen wesentlichen Spielraum lässt; somit kann in diesem Zustand auch von Zwangsbedingungen gesprochen werden, die von der genannten Anordnung herrühren und den Greifarmen keine andere Möglichkeit gibt, als eine im Wesentlichen vollständig gestreckte Form einzunehmen. Wird die Handhabungseinrichtung in Richtung ihrer proximalen Endstellung bewegt und werden somit die Greifarme entsprechend ausgefahren, so bewirkt der gekrümmte Verlauf des mittleren Abschnittes der Greifarme zunächst ein Aufspreizen der Greifarme, zumindest solange der gebogene Verlauf des nach außen gekrümmten mittleren Abschnittes der Greifarme zur Entstehung einer den mittleren Abschnitt der Greifarme nach außen auf die Innenseite der Hülse gerichteten Kraftbeiträgt, und anschließend wieder ein Schließen der Greifarme, spätestens wenn der nach außen gekrümmte mittlere Abschnitt der Greifarme außerhalb der Hülse freiliegt und somit die Greifarme keinerlei von der Hülse verursachten Zwangseinflüssen mehr unterliegen.

Bei einer bevorzugten Weiterbildung ist die Innenseite der Hülse mit in Bewegungsrichtung der Handhabungseinrichtung verlaufenden Führungsnuten versehen, in denen die Greifarme in deren Längsrichtung beweglich angeordnet sind, und die Anordnung so getroffen, dass in der proximalen Endstellung der Handhabungseinrichtung die Greifarme mit ihrem an ihr proximales Ende anschließenden Abschnitt über das proximale Ende des Rohres hinausragen und somit freiliegen.

Für eine sichere Auflage auf die Oberfläche des Körpers weist vorzugsweise die Hülse einen flanschartigen Rand auf.

Bei einer weiteren bevorzugten Ausführung ist die Schlauchhülle als Doppelhülle mit einer Innenhülle und einer die Innenhülle in einem Abstand unter Bildung eines Zwischenraumes umgebenden Außenhülle ausgebildet und an den zwischen Innenhülle und Außenhülle gebildeten Zwischenraum eine Druckluftquelle anschließbar. Durch den Anschluss einer Druckluftquelle lässt sich der Zwischenraum zwischen Innenhülle und Außenhülle aufblasen und unter Druck setzen. Dies hat drei Effekte zur Folge. Um das zu entfernende Organ herum wird ein größerer Raum geschaffen, was die Handhabung der Vorrichtung und insbesondere der Handhabungseinrichtung erleichtert. Des Weiteren wird die Umgebung des zu entfernenden Organs innerhalb des Körpers stabilisiert. Schließlich, dies ist ein besonders wichtiger Punkt, wird das zu entsorgende Organ mit Druck beaufschlagt, der eine Komprimierung des Organs bewirkt, wodurch die Entfernung des Organs durch das Rohr zusätzlich zu dem aufgrund der Saugwirkung dort herrschenden Unterdruck noch beschleunigt und somit begünstigt wird.

Bei dieser Ausführung kann die Schließeinrichtung zum Verschließen des proximalen Endes der Innenhülle und zum Verschließen des proximalen Endes der Außenhülle oder alternativ zum gemeinsamen Verschließen der proximalen Enden der Innenhülle und der Außenhülle ausgebildet sein.

Bei einer weiteren bevorzugten Weiterbildung dieser Ausführung ist die Handhabungseinrichtung zumindest abschnittsweise in dem zwischen Innenhülle und Außenhülle gebildeten Zwischenraum angeordnet. Dadurch wird eine Berührung der Handhabungseinrichtung mit dem zu entsorgenden Organ vermieden und eine sichere Handhabung der Schlauchhülle für die Umschließung des Organs gewährleistet.

Bei einer weiteren bevorzugten Weiterbildung dieser Ausführung ist am proximalen Ende der Schlauchhülle die Innenhülle mit der Außenhülle dichtend verbunden. Diese Weiterbildung ist besonders vorteilhaft, weil die Schließeinrichtung nur in einem einzigen Arbeitsgang das gemeinsame proximale Ende der von der Innenhülle und der Außenhülle gebildeten Schlauchhülle zu verschließen hat. Ein weiterer Vorteil besteht darin, dass bei Anordnung zumindest abschnittsweise in dem zwischen Innenhülle und Außenhülle gebildeten Zwischenraum die Handhabungseinrichtung mit ihrem proximalen Ende von innen in Anlage an das die Innenhülle mit der Außenhülle dichtend verbindende proximale Ende der Schlauchhülle gelangt und sich dadurch die Schlauchhülle mithilfe der Handhabungseinrichtung besonders einfach handhaben lässt. Dies gilt insbesondere bei Verwendung von Greifarmen, über die sich die als Doppelhülle ausgebildete Schlauchhülle nach Art eines Strumpfes überziehen lässt.

Bei einer weiteren besonders bevorzugten Ausführung ist das Rohr als Innenrohr ausgebildet, das von einem Außenrohr in einem Abstand unter Bildung eines Zwischenraumes umgeben ist, ist das Innenrohr gegenüber dem Außenrohr bewegbar und dabei mit einem zu seinem proximalen Ende benachbarten Abschnitt aus dem proximalen Ende des Außenrohres ausziehbar angeordnet, die Schlauchhülle vor Benutzung im Wesentlichen in dem Zwischenraum zwischen Innenrohr und Außenrohr angeordnet und durch Ausziehen des Innenrohres aus dem Außenrohr freilegbar und die Zerkleinerungseinrichtung innerhalb des Innenrohres im Bereich seines proximalen Endes vorgesehen. Somit lässt sich mit dieser Ausführung die Schlauchhülle vor Benutzung in dem Zwischenraum zwischen Innenrohr und Außenrohr 'verstecken' und erst nach Einbringen der Vorrichtung in den Körper durch Ausziehen des Innenrohres aus dem Außenrohr entfalten.

Bei Verwendung der zuvor erwähnten Hülse kann diese bevorzugt am proximalen Ende des Außenrohres angeordnet oder sogar vom Außenrohr selbst gebildet sein.

Des Weiteren kann die Handhabungseinrichtung insbesondere in Abhängigkeit vom Platzbedarf der Vorrichtung entweder an der Außenseite des Innenrohres oder am Außenrohr angeordnet und ausgebildet sein.

Zweckmäßigerweise sollte die Schlauchhülle mit ihrem distalen Ende am Innenrohr befestigt sein, da die Entfernung des zu entsorgenden Organs ja durch das Innenrohr stattfindet.

Vor einem Einsatz der Vorrichtung ist gewöhnlich das Innenrohr in das Außenrohr zurückgezogen und befindet sich auch die Handhabungseinrichtung in einer zurückgezogenen Stellung, in der sie zumindest nicht über das Außenrohr und das Innenrohr hinausragt. Beim Ausziehen des Innenrohres aus dem Außenrohr wird bevorzugt zunächst die Handhabungseinrichtung vom Innenrohr mitgenommen oder die Handhabungseinrichtung im Wesentlichen parallel zum Innenrohr bewegt, wobei die Handhabungseinrichtung die Schlauchhülle mitnimmt und zumindest zu einem größeren Teil aus dem ursprünglich zwischen dem Innenrohr und dem Außenrohr gebildeten Zwischenraum herauszieht und somit vom Außenrohr entfernt, bis das proximale Ende des Innenrohres sich in der Nähe des zu entfernenden Organs befindet oder sogar in Anlage an dieses gelangt ist. Anschließend wird bevorzugt die Handhabungseinrichtung gegenüber dem Innenrohr bewegt, um die von ihr mitgenommene Schlauchhülle an ihrem proximalen Ende zu öffnen oder aufzuweiten und um das zu entfernende Organ herumzuführen oder zu legen. Alternativ oder zusätzlich ist es aber auch denkbar, bei einer Weiterbildung der zuvor erwähnten Ausführung das Innenrohr so auszubilden, dass es beim Ausziehen aus dem Innenrohr die Schlauchhülle mitnimmt und dadurch im Wesentlichen freilegt.

Bei einer Ausbildung der Schlauchhülle als Doppelhülle kann vorzugsweise die Innenhülle am Innenrohr und die Außenhülle am Außenrohr befestigt und die Druckluftquelle an den zwischen Innenrohr und Außenrohr gebildeten Zwischenraum anschließbar sein. Somit lässt sich nicht nur die Druckluftquelle besonders einfach und zugleich sicher außerhalb des Körpers an die Vorrichtung anschließen, sondern die Druckluft auch besonders einfach und zugleich wirkungsvoll in die als Doppelhülle ausgebildete Schlauchhülle im Inneren des Körpers leiten.

Nachfolgend werden bevorzugte Ausführungsbeispiele der Erfindung anhand der beiliegenden Zeichnungen näher erläutert. Es zeigen:
- Fig. 1: schematisch im Längsschnitt eine Vorrichtung zur Entfernung von Organen aus dem menschlichen oder tierischen Körper gemäß einer ersten bevorzugten Ausführung in einem anfänglichen ersten Betriebszustand;
- Fig. 2: schematisch im Längsschnitt die Vorrichtung von Fig. 1 in einem zweiten Betriebszustand, in der sie sich bereits in Berührung mit einem zu entfernenden Organ befindet;
- Fig. 3: schematisch im Längsschnitt in einer vergrößerten, abschnittsweisen Ansicht die Vorrichtung von Fig. 1 in einem dritten Betriebszustand;
- Fig. 4: schematisch im Längsschnitt in einer vergrößerten, ausschnittsweisen Ansicht die Vorrichtung von Fig. 1 in einem vierten Betriebszustand;
- Fig. 5: schematisch im Längsschnitt in einer vergrößerten, ausschnittsweisen Ansicht die Vorrichtung von Fig. 1 in einem fünften Betriebszustand mit nun von einer Schlauchhülle vollständig umschlossenem, zu entfernendem Organ;
- Fig. 6: schematisch im Längsschnitt in einer vergrößerten, ausschnittsweisen Ansicht die Vorrichtung von Fig. 1 in einem sechsten Betriebszustand mit von der Schlauchhülle vollständig umschlossenem, zu entfernendem Organ;
- Fig. 7: schematisch im Längsschnitt in einer vergrößerten, ausschnittsweisen Ansicht die Vorrichtung von Fig. 1 in einem siebten Betriebszustand mit dem nun aus dem Körper im Wesentlichen entfernten Organ;
- Fig. 8: schematisch im Längsschnitt die Vorrichtung von Fig. 1 in einem im Wesentlichen vollständig vom Körper entfernten achten Betriebszustand;
- Fig. 9: schematisch in einer vergrößerten, ausschnittsweisen, perspektivischen Ansicht eine Hülse und die proximalen Enden von zumindest einen Teil einer Handhabungseinrichtung bildenden Greifarmen in einer noch geschlossenen Stellung als Teil einer Vorrichtung zur Entfernung von Organen aus dem menschlichen oder tierischen Körper gemäß einer zweiten bevorzugten Ausführung;
- Fig. 10: im Wesentlichen die gleiche Ansicht wie Fig. 9 mit nun bereits leicht geöffneten Greifarmen;
- Fig. 11: im Wesentlichen die gleiche Ansicht wie Fig. 9 mit im Wesentlichen vollständig gespreizten und somit geöffneten Greifarmen;
- Fig. 12: schematisch in perspektivischer Ansicht zumindest einen Teil einer Handhabungseinrichtung mit Greifarmen als Teil einer Vorrichtung zur Entfernung von Organen aus dem menschlichen oder tierischen Körper gemäß einer dritten bevorzugten Ausführung;
- Fig. 13: in Seitenansicht die Formgebung eines Greifarmes gemäß der ersten Ausführung von Fig.12; und
- Fig. 14: schematisch in perspektivischer Seitenansicht zumindest einen Teil einer Handhabungseinrichtung mit Greifarmen als Teil einer Vorrichtung zur Entfernung von Organen aus dem menschlichen oder tierischen Körper gemäß einer vierten bevorzugten Ausführung, wobei Fig. 14a die Handhabungseinrichtung gemäß der zweiten Ausführung vollständig und Fig. 14b einen Abschnitt im Bereich des proximalen Endes eines inneren Rohres zeigt.

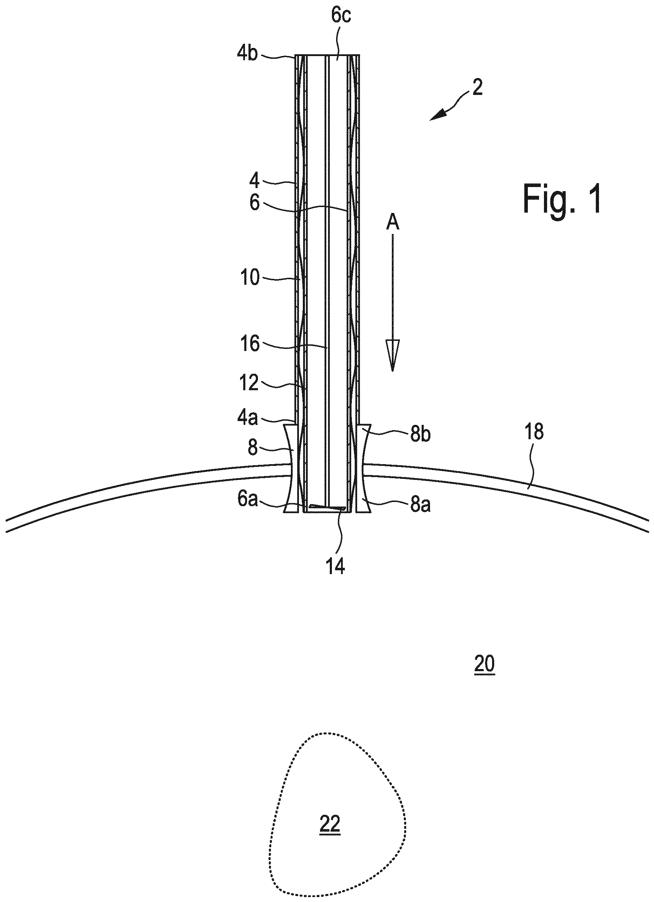

In Fig. 1 ist schematisch im Längsschnitt eine Vorrichtung 2 zur Entfernung von Organen aus dem menschlichen oder tierischen Körper gemäß einem ersten bevorzugten Ausführungsbeispiel in einem anfänglichen ersten Betriebszustand gezeigt.

Die Vorrichtung 2 weist ein äußeres Rohr 4 mit einem offenen proximalen Ende 4a und einem offenen distalen Ende 4b und ein inneres Rohr 6 mit einem offenen proximalen Ende 6a und einem offenen distalen Ende 6b auf. Des Weiteren weist die Vorrichtung 2 im dargestellten Ausführungsbeispiel eine Hülse 8 auf, die ein offenes proximales Ende 8a und ein offenes distales Ende 8b aufweist und eine Verlängerung des äußeren Rohres 4 bildet, indem das äußere Rohr 4 mit seinem proximalen Ende 4a am distalen Ende 8b der Hülse 8 befestigt ist. Das innere Rohr 6 ist gegenüber der Anordnung von äußerem Rohr 4 und Hülse 8 in deren Längsrichtung bewegbar gelagert, wozu entsprechend geeignete Lager- bzw. Führungselemente vorzusehen sind, die in den Figuren jedoch nicht abgebildet sind. Zwischen der Innenseite des äußeren Rohres 4 und der Hülse 8 einerseits und der Außenseite des inneren Rohres 6 andererseits ist ein im Querschnitt ringförmiger Zwischenraum 10 gebildet, der in dem anfänglichen ersten Betriebszustand von Fig. 1 eine Schlauchhülle 12 aufnimmt. Des Weiteren enthält die Vorrichtung 2 im dargestellten Ausführungsbeispiel eine Zerkleinerungseinrichtung, die ein im Bereich des proximalen Endes 8a der Hülse 8 angeordnetes Messer aufweist, das im dargestellten Ausführungsbeispiel als um die Längsachse des inneren Rohres 6 rotierbar gelagertes Drehmesser aufweist, das am Ende einer Drehwelle 16 sitzt, deren Drehachse mit der Längsachse des inneren Rohres 6 zusammenfällt und von einem nicht dargestellten Motor in Rotation versetzt wird. Wie Fig. 1 ferner erkennen lässt, sind das äußere Rohr 4, das innere Rohr 6 und die Hülse 8 im dargestellten Ausführungsbeispiel im Wesentlichen konzentrisch zueinander angeordnet, so dass deren Längsachsen im Wesentlichen zusammenfallen.

Die Hülse 8 dient zum Einsetzen der Vorrichtung 2 in die Haut 18 eines menschlichen oder tierischen Körpers 20 und gleichzeitig zur Fixierung der Vorrichtung 2 in Richtung auf ein aus dem Körper 20 zu entfernendes Organ 22, wie Fig. 1 ebenfalls erkennen lässt. Bei dem in Fig. 1 gezeigten anfänglichen ersten Betriebszustand ist somit die Vorrichtung 2 mit Hilfe der Hülse 8 bereits auf der Haut 18 des Körpers 20 angeordnet bzw. in die Haut 18 des Körpers 20 eingesetzt, befindet sich jedoch noch nicht weiter in Funktion.

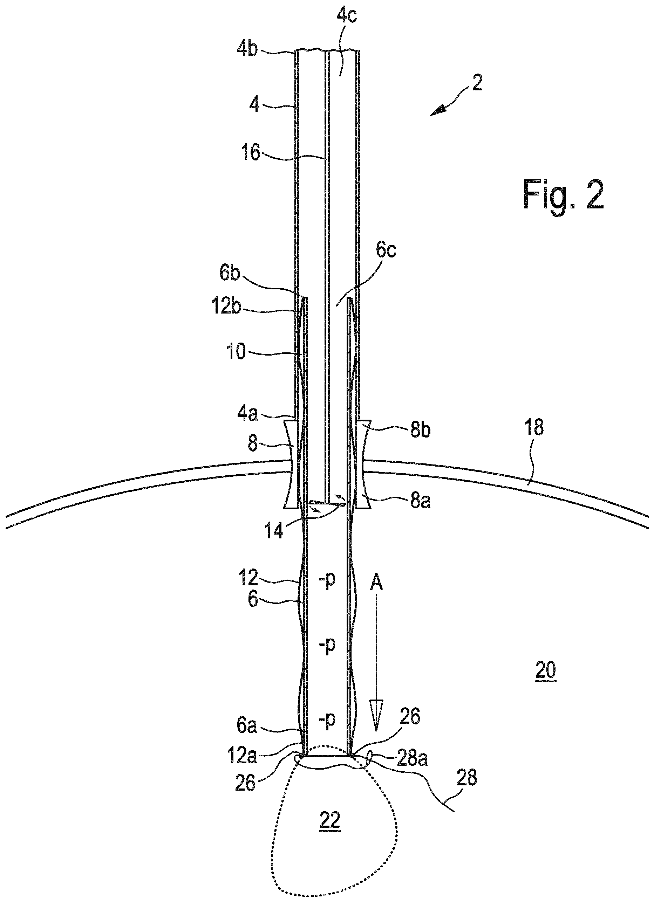

In Fig. 2 ist die Vorrichtung 2 in einem zweiten Betriebszustand gezeigt. In diesem Zustand ist in Richtung des Pfeils A das innere Rohr 6 gegenüber dem äußeren Rohr 4 und der Hülse 8 bewegt und dabei aus dem proximalen Ende 8a der Hülse 8 ausgezogen und in Richtung auf das zu entfernende Organ 22 tiefer in den Körper 20 eingeführt worden, bis das proximale Ende 6a des inneren Rohres 6 in Berührung mit dem zu entfernenden Organ 22 gelangt. Jedoch verbleibt das innere Rohr 6 mit seinem distalen Ende 6b und einem sich daran anschließenden Abschnitt weiterhin innerhalb des äußeren Rohres 4 und der Hülse 8, während sich das innere Rohr 6 mit dem übrigen, zu seinem proximalen Ende 6a benachbarten Abschnitt aus der Hülse 8 in den Körper 20 erstreckt und demnach mit diesem Abschnitt innerhalb des Körpers 20 freiliegt. Gleiches gilt auch für die Schlauchhülle 12, die während der Bewegung aus dem äußeren Rohr 4 und der Hülse 8 heraus vom inneren Rohr 6 mitgenommen wird, sodass sie mit ihrem distalen Ende 12b weiterhin innerhalb des verbliebenen Zwischenraumes 10 zwischen dem äußeren Rohr 4 und der Hülse 8 einerseits und dem inneren Rohr 6 im Abschnitt benachbart zum distalen Ende 6b des inneren Rohres 6 angeordnet ist, im Übrigen jedoch freiliegt und sich ihr proximales Ende 12a, das vom inneren Rohr 6 während dessen Bewegung mitgenommen wurde, benachbart zum Organ 22 befindet. An das offene distale Ende 4b des äußeren Rohres 4 ist eine in den Figuren nicht dargestellte Unterdruck- bzw. Saugeinrichtung angeschlossen, um in dem Hohlraum 4c des äußeren Rohres 4b und in dem mit dem Hohlraum 4c des äußeren Rohres 4 über das offene distale Ende 6b des inneren Rohres 6 kommunizierenden Hohlraum 6c des inneren Rohres 6 einen Unterdruck zu erzeugen, der in Fig. 2 mit "-p" schematisch angedeutet ist. Aufgrund des Unterdruckes innerhalb des Hohlraumes 6c des inneren Rohres 6 entsteht am proximalen Ende 6a des inneren Rohres 6 eine Saugwirkung, wodurch das Organ 22 an das proximale Ende 6a des inneren Rohres 6 gesogen wird. Auf diese Weise wird aus einer zunächst losen Berührung des proximalen Endes 6a des inneren Rohres 6 mit dem zu entfernenden Organ 22 eine Fixierung des Organs 22 am proximalen Ende 6a des inneren Rohres 6.

Wie Fig. 2 ferner schematisch erkennen lässt, sind im dargestellten Ausführungsbeispiel am proximalen Ende 12a der Schlauchrolle 12 Ösen 26 befestigt, durch die ein Draht oder Faden 28 ziehbar ist, der an seinem einen Ende mit einer Schlaufe 28a versehen ist. In Fig. 2 ist der Faden 28 in einem Zustand erkennbar, in dem er bereits durch die Öse 26 und die Schlaufe 28a gezogen ist. Der Faden 28 ist gewöhnlich bereits vor Benutzung der Vorrichtung 2 entsprechend konfektioniert, also durch die Öse 26 am proximalen Ende 12a der Schlauchhülle 12 und durch die an seinem einen Ende vorgesehene Schlaufe 28a in einem losen Zustand gezogen bzw. geführt. Demnach liegen in dem anfänglichen ersten Betriebszustand gemäß Fig. 1 auch der Faden 28 zusammen mit den Ösen 26 und der Schlaufe 28a im Zwischenraum 10 zwischen dem äußeren Rohr 4 und der Hülse 8 einerseits und dem inneren Rohr 6 andererseits, und zwar gewöhnlich im Bereich des proximalen Endes 4a des äußeren Rohres 4 oder der Hülse 8, was jedoch in Fig. 1 nicht dargestellt ist. In dem zweiten Betriebszustand der Vorrichtung 2 gemäß Fig. 2 und somit im ausgefahrenen Zustand des inneren Rohres 6 kann die nachfolgend noch im Einzelnen näher beschriebene Bedienung des Fadens 28 auf unterschiedliche Weise erfolgen. Beispielsweise kann der Faden 28 entlang des inneren Rohres 6 durch den zwischen dem äußeren Rohr 4 und der Hülse 8 einerseits und dem zum distalen Ende 6b benachbarten Abschnitt des inneren Rohres 6 andererseits verbliebenen Zwischenraum 10 und anschließend durch den Hohlraum 4c des äußeren Rohres 4 nach außen geführt sein und von dort bedient werden. Alternativ ist es aber auch denkbar, den Faden 28 mithilfe eines in den Figuren nicht gezeigten Endoskops zu bedienen, das bevorzugt durch eine vorhandene Körperöffnung in den Körper 20 eingeführt wird. Außerdem ist es anstelle der zuvor erwähnten Konfektionierung bzw. Handhabung auch theoretisch zumindest denkbar, den Faden 28 erst nachträglich innerhalb des Körpers 20 durch die Ösen 26 und die Schlaufe 28a zu ziehen, was in der Regel dann nur mithilfe eines ebenfalls in den Figuren nicht gezeigten Endoskops durchzuführen ist.

Im Übrigen sei an dieser Stelle der guten Vollständigkeit halber noch darauf hingewiesen, dass spätestens nach Erreichen des in Fig. 2 dargestellten zweiten Betriebszustandes der Vorrichtung 2 das zu entfernende Organ 22 innerhalb des Körpers 20 von seiner Umgebung abgetrennt und somit gelöst sein sollte, wozu bevorzugt gleichfalls ein ebenfalls in den Figuren nicht gezeigtes Endoskop mit einem Schneidmesser zu verwenden ist.

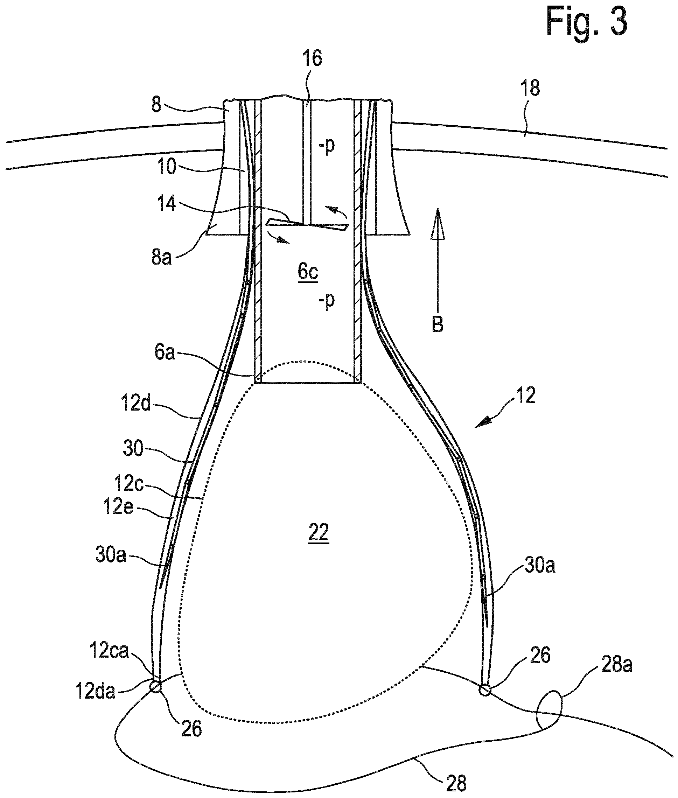

Fig. 3 lässt weitere Einzelheiten der Vorrichtung 2 gemäß der ersten Ausführung erkennen. So ist die Schlauchhülle 12 als Doppelhülle mit einer Innenhülle 12c und einer Außenhülle 12d ausgeführt, wobei zwischen der Innenhülle 12c und der Außenhülle 12d ein Zwischenraum 12e gebildet wird. Zwar ist zumindest in den in den Figuren 1 bis 3 gezeigten Betriebszuständen die Schlauchhülle 12 an ihrem proximalen Ende 12a offen; jedoch ist der Zwischenraum 12e zwischen der Innenhülle 12c und der Außenhülle 12d am proximalen Ende 12a der Schlauchhülle 12 geschlossen, indem das proximale Ende 12ca der Innenhülle 12c und das proximale Ende 12da der Außenhülle 12d über den gesamten Umfang des proximalen Endes 12a der Schlauchhülle 12 miteinander verbunden sind.

Ferner lässt Fig. 3 als weitere Komponente der Vorrichtung 2 zumindest einen Teil einer Handhabungseinrichtung in Form von Greifarmen 30 erkennen, die im dargestellten Ausführungsbeispiel eine längliche Stabform besitzen und in Richtung der Längserstreckung des inneren Rohres 6 orientiert sind. Die Greifarme 30 sind innerhalb des Zwischenraumes 10 angeordnet. Ferner sind die Greifarme 30 in Richtung ihrer Längserstreckung sowohl gegenüber dem äußeren Rohr 4 und der Hülse 8 einerseits als auch gegenüber dem inneren Rohr 6 bewegbar, wozu die Handhabungseinrichtung entsprechend geeignete, jedoch in den Zeichnungen nicht dargestellte Lager- und Führungsmittel sowie ebenfalls in den Zeichnungen nicht dargestellte Aktuatoren und/oder Handhabungs- bzw. Antriebsmittel aufweist. Bevorzugt sind die Greifarme 30 an der Außenseite des inneren Rohres 6 in dessen Längsrichtung bewegbar gelagert, sodass beim Ausfahren des inneren Rohres 6 aus der Hülse 8 beim Übergang von dem anfänglichen ersten Betriebszustand der Vorrichtung 2 gemäß Fig. 1 in den zweiten Betriebszustand der Vorrichtung 2 gemäß Fig. 2 die Greifarme 30 zumindest zunächst vom inneren Rohr 6 mitgenommen werden. Alternativ ist es grundsätzlich aber auch denkbar, die Greifarme 30 an der Innenseite des äußeren Rohres 4 in dessen Längsrichtung bewegbar zu lagern. Im anfänglichen ersten Betriebszustand der Vorrichtung 2 sind auch die Greifarme 30 innerhalb des Zwischenraumes zwischen dem äußeren Rohr 4 und der Hülse 8 einerseits und dem inneren Rohr 6 untergebracht, was jedoch in den Figuren 1 und 2 nicht erkennbar dargestellt ist.

Wie Fig. 3 des Weiteren erkennen lässt, sind die Greifarme 30 zumindest mit ihrem benachbart zu ihrem proximalen Ende 30a gelegenen Abschnitt zwischen der Innenhülle 12c und der Außenhülle 12d und somit innerhalb des zwischen der Innenhülle 12c und der Außenhülle 12d gebildeten Zwischenraumes 12e innerhalb der Schlauchhülle 12 angeordnet, sodass in anderen Worten die als Doppelhülle ausgebildete Schlauchhülle 12 nach Art eines Strumpfes die Greifarme 30 in sich aufnimmt. Hierzu ist die Innenhülle 12c an der Außenseite des inneren Rohres 6, bevorzugt im Bereich seines proximalen Endes 6a, und die Außenhülle 12d an der Innenseite der Hülse 8 oder des äußeren Rohres 4 befestigt ist, sodass der zwischen dem äußeren Rohr 4 und der Hülse 8 einerseits und dem inneren Rohr 6 andererseits gebildete Zwischenraum 10 mit dem innerhalb der Schlauchhülle 12 zwischen der Innenhülle 12c und der Außenhülle 12d gebildeten Zwischenraum 12e kommuniziert. Eine solche Anordnung sorgt nun bei dieser Ausführung dafür, dass die Schlauchhülle 12 von den Greifarmen 30 geführt wird, und zwar nicht nur beim Austreten aus dem Zwischenraum 10 zwischen dem äußeren Rohr 4 und der Hülse 8 einerseits und dem inneren Rohr 6 andererseits während der Ausfahrbewegung des inneren Rohres 6 aus der Hülse 8 in Richtung des in den Fign. 1 und 2 gezeigten Pfeils A, sondern auch für eine Relativbewegung der Schlauchhülle 12 gegenüber dem inneren Rohr 6, insbesondere um die Schlauchhülle 12 über das proximale Ende 6a des inneren Rohres 6 hinaus in Richtung auf das zu entfernende Organ 22 zu führen, und zwar ebenfalls im Wesentlichen in Richtung des in den Fign. 1 und 2 abgebildeten Pfeils A.

Die Greifarme 30 sind jedoch nicht nur in ihrer Längsrichtung bewegbar, sondern, wie Fig. 3 ebenfalls erkennen lässt, in Querrichtung bewegbar und somit spreizbar. Denn, wie Fig. 3 ebenfalls zu entnehmen ist, ist das zu entfernende Organ 22 in der Regel breiter als der Durchmesser des inneren Rohres 6. Somit ist auch eine nach außen gerichtete Spreizbewegung der Greifarme 30 erforderlich, um während ihrer gleichzeitigen Bewegung in Längsrichtung das zu entfernende Organ 22 umfassen und dabei die Schlauchhülle 12 um das Organ 22 herumlegen zu können. Hierzu bestehen die Greifarme 30 aus einer Vielzahl von gelenkig miteinander verbundenen Gliedern, wie Fig. 3 schematisch erkennen lässt, und/oder sind die Greifarme 30 in Richtung quer zu ihrer Längserstreckung flexibel oder elastisch ausgebildet. Die Spreizbewegung nach außen kann beispielsweise durch in den Figuren nicht dargestellte Mikroantriebe und/oder Federn und/oder durch ein besonderes Zusammenwirken von Form und Elastizität erzeugt werden.

Die Relativbewegung zwischen den Greifarmen 30 und dem inneren Rohr 6 kann noch dadurch unterstützt werden, dass während der Spreizbewegung der Greifarme 30 das innere Rohr 6 nun einer entgegengesetzten Bewegung wieder zurück in Richtung auf die Hülse 8 unterworfen wird und somit beginnt, wieder in die Hülse 8 und das äußere Rohr 4 eingefahren zu werden, wie in Fig. 3 durch den Pfeil B angedeutet ist. Dadurch wird das Organ 22 sozusagen zwischen die aufgespreizten Greifarme 30 verbracht.

Während dieses Bewegungsablaufes umschließen die ursprünglich aufgespreizten Greifarme 30 das zu entfernende Organ 22, indem die Greifarme mit ihren proximalen Enden 30a nun eine Bewegung aufeinanderzu ausführen. Auch diese nun zur Spreizbewegung entgegengesetzte Bewegung lässt sich beispielsweise durch die bereits erwähnten und in den Figuren nicht dargestellten Mikroantriebe oder Federn oder durch eine kombinierte Wirkung aus spezieller Formgebung und Elastizität bewirken.

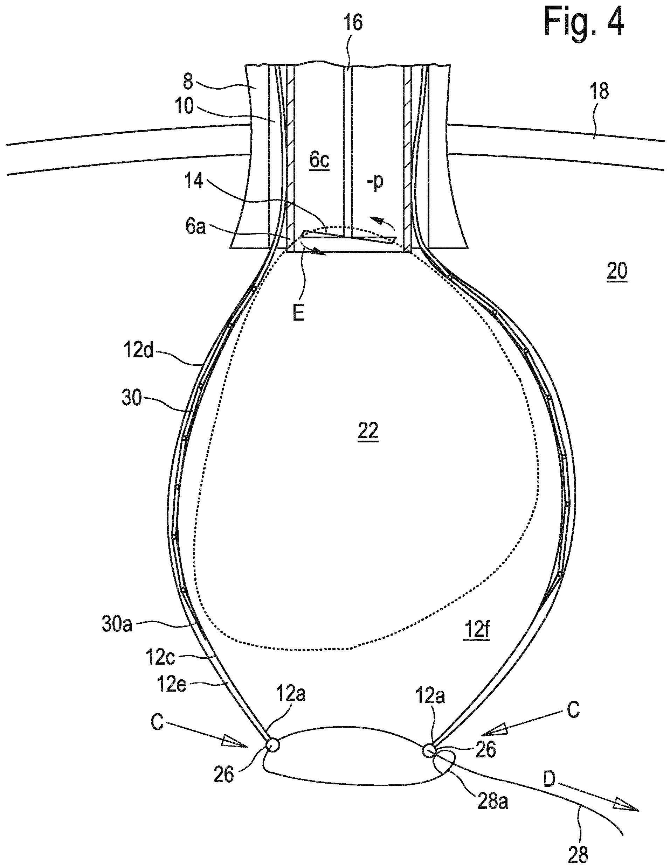

Mit zunehmender Bewegung der proximalen Enden 30a jeweils gegenüberliegender Greifarme 30 in Richtung des Pfeils C, wie Fig. 4 erkennen lässt, umschließen die Greifarme 30 das zu entfernende Organ 22, sodass diese Bewegung auch als Schließbewegung bezeichnet werden kann. Diese Schließbewegung hat nun zur Folge, dass durch die Greifarme 30 die Schlauchhülle 12 um das zu entfernende Organ 22 gelegt wird, das nach wie vor aufgrund der Saugwirkung im Hohlraum 6c des inneren Rohres 6 an dem proximalen Ende 6a des inneren Rohres 6 hängt. In diesem vierten Betriebszustand, wie er in Fig. 4 gezeigt ist, ist des Weiteren das innere Rohr 6 wieder so weit in die Hülse 8 eingefahren, dass das proximale Ende 6a des inneren Rohres 6 etwa auf der Höhe des proximalen Endes 8a der Hülse 8 liegt. Wie bereits zuvor erwähnt, begünstigt die Aufwärtsbewegung des inneren Rohres 6 mit dem daran hängenden Organ 22 die Schließbewegung der Greifarme und das dadurch bewirkte Umhüllen des Organs 22 mit der Schlauchhülle 12.

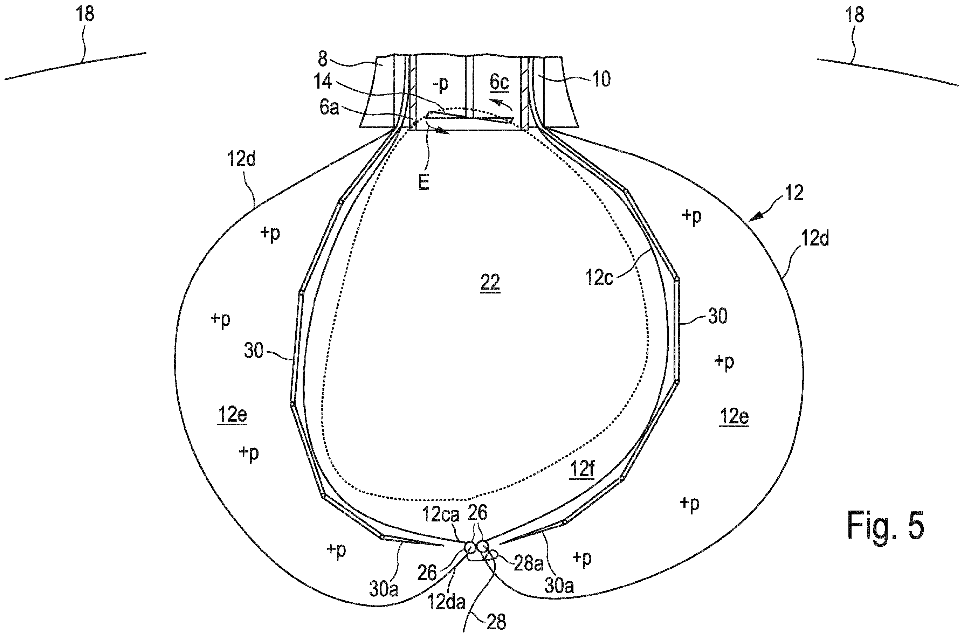

Entgegen den Darstellungen in den Figuren 3 und 4 ist es während der anhand der Figuren 3 und 4 beschriebenen Bewegungsabläufe gewöhnlich so, dass die Greifarme 30 mit ihrem proximalen Ende 30a im Wesentlichen an der Innenseite des geschlossenen proximalen Endes 12a der als Doppelhülle ausgebildeten Schlauchhülle 12 anliegen, was auch die Führung der Schlauchhülle 12 im Bereich ihres proximalen Endes 12a durch die Greifarme 30 erleichtert, wie im Übrigen in Fig. 5 erkennbar ist.

In dem dritten Betriebszustand der Vorrichtung 2 gemäß Fig. 3 und auch in dem vierten Betriebszustand der Vorrichtung 2 gemäß Fig. 4 und den zugehörigen, zuvor beschriebenen Bewegungsabläufen der Greifarme 30 bleibt der durch die am proximalen Ende 12a der Schlauchhülle 12 angeordneten Ösen 26 und die Schlaufe 28a geführte Faden 28 in einem sehr losen Zustand, um die beschriebenen Bewegungsabläufe und insbesondere die Umhüllung des Organs 22 mit der Schlauchhülle 12 nicht zu behindern.

Der letzte Teil der Bewegung der proximalen Enden 30a der Greifarme 30 aufeinander zu in Richtung des Pfeils C wird dann von dem Faden 28 unterstützt oder sogar ausschließlich übernommen, indem nun der Faden 28 einer Zugbewegung in Richtung des in Fig. 4 gezeigten Pfeils D unterworfen wird. Dadurch lässt sich dann die Schlauchhülle 12 an ihrem proximalen Ende 12a verschließen, wie Fig. 5 erkennen lässt, in der ein fünfter Betriebszustand der Vorrichtung 2 abgebildet ist. Somit bildet nun die Schlauchhülle 12 einen von ihrer Innenhülle 12c begrenzten, geschlossenen Hohlraum 12f, in dem das zu entfernende Organ 22 enthalten ist. Mit anderen Worten hat nun die Schlauchhülle 12 die Form eines Beutels, der das zu entfernende Organ 22 aufnimmt.

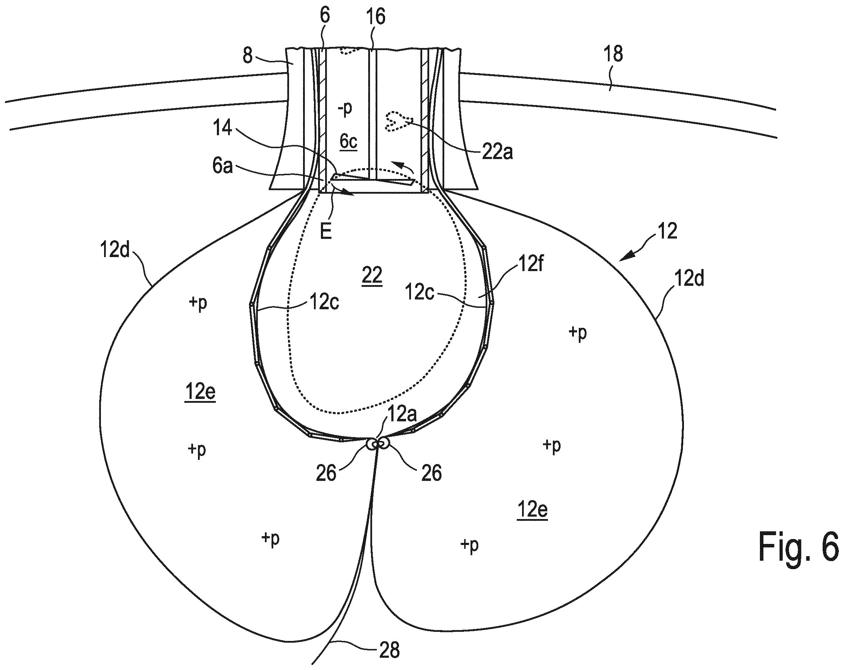

Ferner ist in dem in Fig. 5 abgebildeten fünften Betriebszustand der Vorrichtung 2 Druckluft durch den Zwischenraum 10 zwischen dem äußeren Rohr 4 und der Hülse 8 einerseits und dem inneren Rohr 6 andererseits (Fig. 1) und somit auch in den sich an diesen Zwischenraum 10 anschließenden Zwischenraum 12e zwischen der Innenhülle 12c und der Außenhülle 12d eingeblasen worden, wozu im Bereich des distalen Endes 4b des äußeren Rohres 4 eine in den Figuren nicht dargestellte Druckluftquelle an den Zwischenraum 10 angeschlossen wird. Dies hat zur Folge, dass der Zwischenraum 12e zwischen der Innenhülle 12c und der Außenhülle 12d aufgeblasen wird, wobei der in diesem Zwischenraum 12e herrschende Überdruck in den Figuren 5 und 6 mit "+p" angedeutet ist. Dieser Überdruck führt einerseits zu einer Druckbeaufschlagung des zu entfernenden Organs 22 über die Innenhülle 12c und andererseits zum Aufblasen der Außenhülle 12d.

Durch den im Hohlraum 6c des inneren Rohres 6 herrschenden Unterdruck wird das zu entsorgende Organ 22 nicht nur an das proximale Ende 6a des inneren Rohres 6 angesogen, sondern auch in dieses hineingezogen und gelangt dabei in den Wirkungsbereich des sich in Richtung des Pfeils E rotierenden Drehmessers 14. Durch die zusätzliche Druckbeaufschlagung über die Innenhülle 12c aufgrund des dahinter im Zwischenraum 12e herrschenden Überdruckes wird das zu entfernende Organ 22 noch stärker in das proximale Ende 6a des inneren Rohres 6 gepresst, wobei gleichzeitig eine Komprimierung des Organs 22 stattfindet. Dieser Effekt wird noch dadurch verstärkt, dass nunmehr das Organ 22 durch das Drehmesser 14 in Einzelteile zerkleinert wird, die sich dann leichter durch das innere Rohr 6 aufsaugen lassen. Dieser sechste Betriebszustand der Vorrichtung 2 ist in Fig. 6 abgebildet, in der beispielhaft auch ein durch das Drehmesser 14 vom Organ 22 abgeschnittenes Teil 22a des Organs 22 innerhalb des Hohlraums 6c des inneren Rohres 6 dargestellt ist.

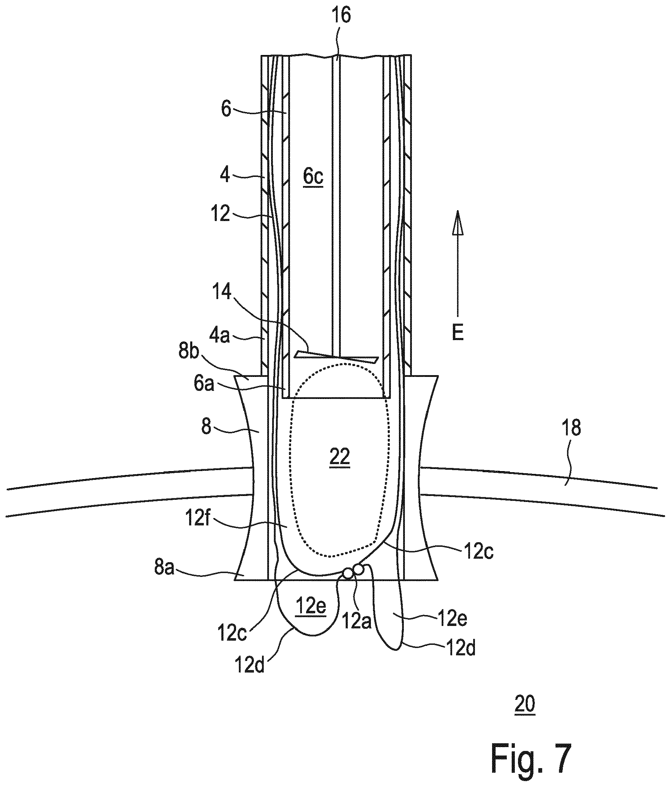

Wenn dann das zu entfernende Organ 22 durch Zusammenwirken von Saugdruck am proximalen Ende 6a des inneren Rohres 6, über die Innenhülle 12c der Schlauchhülle 12 auf das Organ 22 ausgeübtem Überdruck und Drehmesser 14 auf eine Breite komprimiert ist, die zumindest kleiner als der Innendurchmesser der Hülse 8 und des sich daran anschließenden äußeren Rohres 4 ist, kann das restliche, so geschrumpfte Organ 22 in einem weiteren Arbeitsschritt vollständig in einem einzigen Stück aus dem Körper 20 entfernt werden. Der diesbezügliche siebte Betriebszustand der Vorrichtung 2 ist in Fig. 7 abgebildet. In diesem Betriebszustand wird dann der Überdruck in dem Zwischenraum 12e zwischen der Innenhülle 12c und der Außenhülle 12d der doppelwandigen Schlauchhülle 12 abgeschaltet bzw. beseitigt und dieser Zwischenraum 12e durch den Zwischenraum 10 zwischen dem äußeren Rohr 4 und der Hülse 8 einerseits und dem inneren Rohr 6 andererseits im Bereich des distalen Endes 4b des äußeren Rohres 4 entlüftet, wodurch sowohl die Innenhülle 12c als auch die Außenhülle 12d in einen entspannten Zustand gelangt. Während die Innenhülle 12c weiterhin die Funktion eines das restliche Organ 22 aufnehmenden Beutels übernimmt und somit weiterhin von dem Gewicht des restlichen Organs 22 beaufschlagt wird, befindet sich die Außenhülle 12d nun in einem völlig unbeaufschlagten und dadurch losen und schlaffen Zustand, wie Fig. 7 erkennen lässt.

Grundsätzlich ist es denkbar, das Organ 22 auch in seinem geschrumpften Zustand, wie in Fig. 7 abgebildet, mithilfe des Drehmessers 14 weiter zu zerkleinern und die Einzelteile dann durch den Hohlraum 6c des inneren Rohres 6 abzusaugen. In dem in Fig. 7 abgebildeten siebten Betriebszustand ist es aber so, dass auf eine weitere Zerkleinerung durch das Drehmesser 14 verzichtet wird, sondern das innere Rohr 6 mit dem an dessen proximalen Ende 6a weiterhin fixierten restlichen Organ 22 nun vollständig aus dem Körper 20 und auch der Hülse 8 heraus in das äußere Rohr 4 in Richtung des in Fig. 7 abgebildeten Pfeils E bewegt wird. Da dann ein weiteres Zerschneiden des Organs 22 durch das Drehmesser 14 nicht mehr erforderlich ist, wird während dieser Rückziehbewegung des inneren Rohres 6 gleichzeitig die das Drehmesser 14 halternde Drehwelle 16 aus der Hülse 8 heraus in Richtung auf das distale Ende 4b des äußeren Rohres 4 bewegt, wobei die Bewegung der Drehwelle 16 und des inneren Rohres 6 in Richtung des Pfeils E auch synchron erfolgen kann. Des Weiteren ist während dieser Bewegung das Drehmesser 14 nicht mehr aktiv, sodass die Drehwelle 16 in Drehrichtung stillsteht, also einer Rotation nicht mehr unterworfen wird. Somit ist bei dieser Ausführung die das Drehmesser 14 und die Drehwelle 16 aufweisende Zerkleinerungseinrichtung so ausgestaltet, dass sie zwischen einer unteren Arbeitsstellung, in der sich das Drehmesser 14 im Bereich des proximalen Endes 8a der Hülse 8 und dabei nach wie vor innerhalb des inneren Rohres 6 befindet, wie in den Figuren 1 bis 6 gezeigt ist, und einer oberen Ruhestellung entfernt von der Hülse 8 in Richtung auf das distale Ende 4b des äußeren Rohres 4, wobei es sich bevorzugt weiterhin innerhalb des inneren Rohres 6 befindet, bewegbar gelagert ist; die zugehörigen Lager- und Führungselemente sowie ein hierfür ggf. noch erforderlicher Antrieb sind in den Figuren nicht abgebildet.

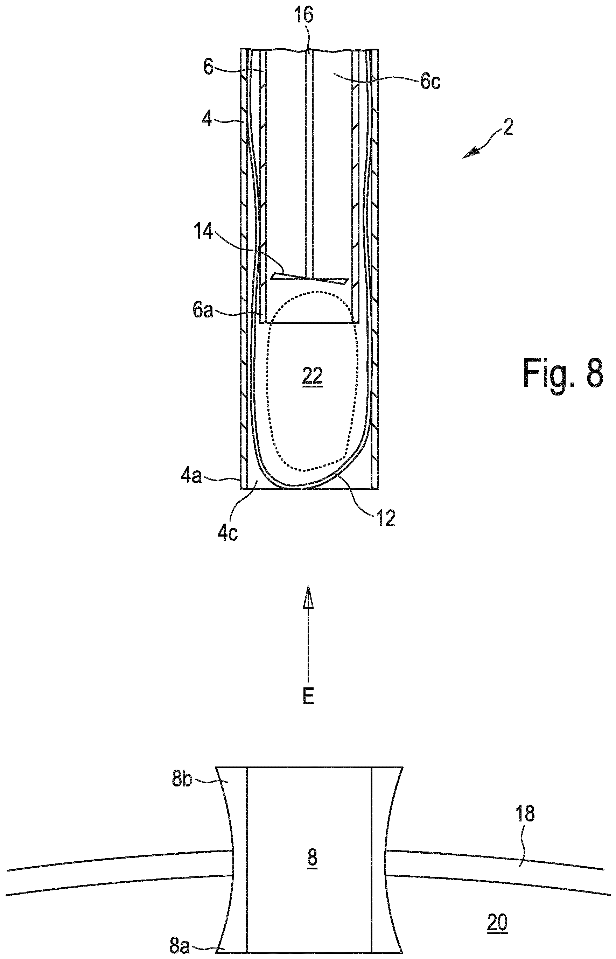

Fig. 8 zeigt die Vorrichtung 2 nun in einem achten Betriebszustand, in der das äußere Rohr 4 mit dem darin wieder aufgenommenen inneren Rohr 6 und dem in der Schlauchhülle 12 enthaltenen entfernten Organ 22 aus der noch in der Haut 18 verbliebenen Hülse 8 herausgezogen ist. Anschließend wird das restliche Organ 22 aus der Vorrichtung 2 entnommen und in gleiche Weise wie die zuvor abgeschnittenen Einzelteile 22a (Fig. 6) entsprechend entsorgt.

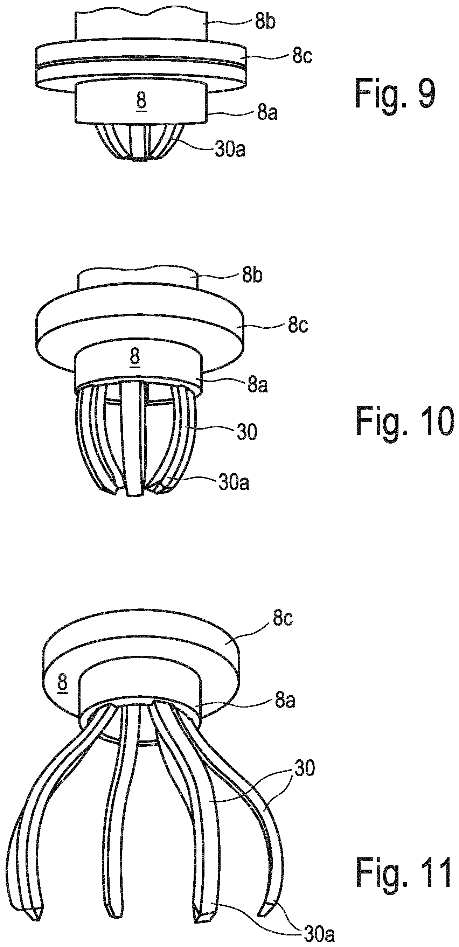

In Fig. 9 sind schematisch in einer vergrößerten, ausschnittsweisen, perspektivischen Ansicht lediglich die Hülse 8 und die proximalen Enden 30a der Greifarme in einem noch geschlossenen Zustand gezeigt. Im Gegensatz zu den Darstellungen in den Figuren 1 bis 8 ist in der Abbildung von Fig. 9 die Hülse 8 noch mit einem umlaufenden Flansch 8c versehen, der beim Einsetzen der Hülse 8 in die Haut 18 des Körpers 20 auf der Haut 18 zu liegen kommt und auf diese Weise eine einfachere und zugleich wirkungsvollere Fixierung der Vorrichtung 2 auf der Haut 18 ermöglicht.

Fig. 10 zeigt im Wesentlichen die gleiche Ansicht wie Fig. 9, wobei gegenüber Fig. 9, die die Greifarme nicht nur mit ihren proximalen Enden 30a in einem geschlossenen Zustand, sondern zugleich auch in einer im Wesentlichen vollständig in der Hülse 8 zurückgezogenen Stellung zeigt, die Greifarme 30 nun in Richtung auf das in Fig. 10 nicht dargestellte zu entfernende Organ weiter ausgefahren und auch schon bereits leicht geöffnet sind.

Fig. 11 zeigt im Wesentlichen die gleiche Ansicht wie Fig. 9 mit nunmehr im Wesentlichen vollständig gespreizten und somit geöffneten Greifarmen 30, wobei dieser Zustand etwa dem in Fig. 3 abgebildeten dritten Betriebszustand der Vorrichtung 2 entspricht.

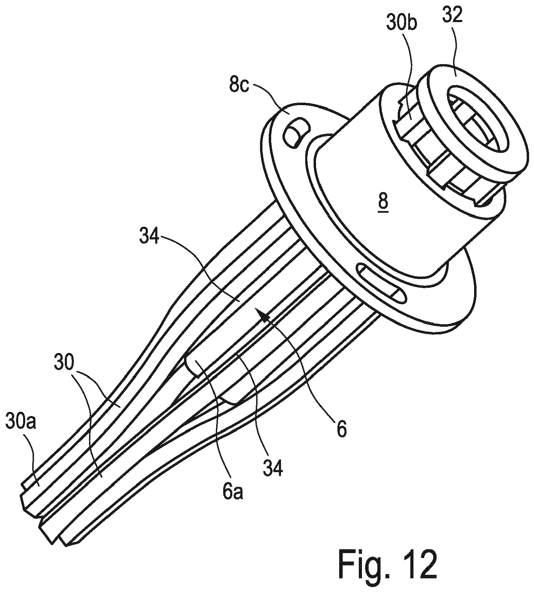

Fig. 12 zeigt schematisch in perspektivischer Ansicht eine Einheit aus innerem Rohr 6, Hülse 8 und Greifarmen 30 als Teil einer Vorrichtung zur Entfernung von Organen aus dem menschlichen oder tierischen Körper gemäß einer weiteren bevorzugten Ausführung. Dabei ist der besseren Übersichtlichkeit halber von dem inneren Rohr 6 nur der zu dessen proximalem Ende 6a benachbarte und aus der Hülse 8 in den Körper 20 ragende und somit freiliegende Abschnitt abgebildet. Ferner bilden die Greifarme 30 wiederum zumindest einen Teil einer im Übrigen nicht dargestellten Handhabungseinrichtung. Wie bei der in den Figuren 9 bis 11 dargestellten Ausführung weist auch bei der in Fig. 12 gezeigten Ausführung die Hülse 8 einen umlaufenden Flansch 8c auf.

Des Weiteren sind in der Ausführung von Fig. 12 die Greifarme 30 an ihren distalen Enden 30b über ein ringförmiges Verbindungselement 32 miteinander verbunden, wodurch die Greifelemente 30 gemeinsam und synchron in Längsrichtung des inneren Rohres 6 bewegbar sind. Das ringförmige Verbindungselement 32 ist somit ebenfalls Teil der im Übrigen nicht dargestellten Handhabungseinrichtung und ist bevorzugt gekoppelt mit einem geeigneten Antrieb, der eine weitere Komponente der erwähnten Handhabungseinrichtung bildet und in den Figuren jedoch nicht dargestellt ist.

Außerdem ist bei der Ausführung gemäß Fig. 12 die Außenseite des inneren Rohres 6 mit in dessen Längsrichtung verlaufenden Führungsnuten 34 versehen, in denen die Greifarme 30 in deren Längsrichtung beweglich aufgenommen sind.

Schließlich sind die Greifarme 30 zwischen der Innenseite der Hülse 8 und der Außenseite des inneren Rohres 6 angeordnet und befinden sich im Wesentlichen in berührender Anlage an der Innenseite der Hülse 8 einerseits und an der Außenseite des inneren Rohres 6 andererseits.

Wie Fig. 12 ferner erkennen lässt, in der die Greifarme 30 in einem entspannten Zustand gezeigt sind, weisen die proximalen Enden 30a von jeweils einander gegenüberliegenden Greifarmen 30 einen Abstand voneinander auf, der kleiner als der Durchmesser des inneren Rohres 6 ist. Ferner weisen die Greifarme 30 gemäß Fig. 12 zwischen ihren proximalen und distalen Enden 30a, 30b einen mittleren Abschnitt auf, der in Bezug auf das innere Rohr 6 nach außen gekrümmt ist, sodass zumindest an ihrer in radialer Richtung am weitesten außen liegenden Stelle der Abstand der mittleren Abschnitte von jeweils einander gegenüberliegenden Greifarmen 30 größer als der Durchmesser des inneren Rohres 6 ist.

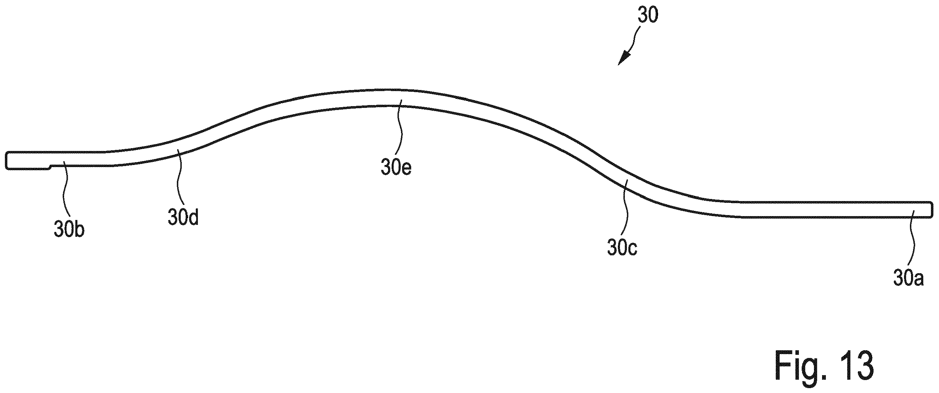

Von den in Fig. 12 abgebildeten Greifarmen 30 ist in Fig. 13 ein einzelner Greifarm 30 in Seitenansicht gezeigt, sodass man aus Fig. 13 den Verlauf bzw. die Formgebung des Greifarmes 30 gut erkennen kann. Gemäß dem in Fig. 13 dargestellten Ausführungsbeispiel weist der Greifarm 30 benachbart zu seinem proximalen Ende 30a einen ersten nach außen gekrümmten Abschnitt 30c und benachbart zu seinem distalen Ende 30b einen zweiten nach außen gekrümmten Abschnitt 30d auf, wobei die beiden nach außen gekrümmten Abschnitte 30c, 30d über den mittleren Abschnitt 30e miteinander verbunden sind, welcher bogenförmig ausgestaltet ist. Des Weiteren ist in Fig. 13 erkennbar, dass die proximalen und distalen Enden 30a, 30b etwa fluchtend zueinander ausgerichtet sind. Schließlich besteht der Greifarm 30 aus elastischem Material, vorzugsweise Kunststoff.

Bei dem in den Figuren 12 und 13 dargestellten Ausführungsbeispiel sind in der zurückgezogenen distalen Endstellung der Handhabungseinrichtung, in der sich die Greifarme 30 in einer sog. Ausgangsposition befinden, innerhalb des Zwischenraumes 10 zwischen dem äußeren Rohr 4 und der Hülse 8 einerseits und dem inneren Rohr 6 andererseits (Fig. 1) zwangsweise in einen im Wesentlichen vollständig gestreckten Zustand verbracht und somit im Wesentlichen vollständig gestreckt, da die radiale Öffnungsweite des Zwischenraumes 10 nur unwesentlich größer als die radiale Dicke der Greifarme 30 ist und der Zwischenraum 10 zwischen der Außenseite des inneren Rohres 6 einerseits und der Innenseite des äußeren Rohres 4 und der Hülse 8 andererseits keinen wesentlichen Spielraum lässt. Somit kann in diesem Zustand auch von Zwangsbedingungen gesprochen werden, die von der genannten Anordnung herrühren und den Greifarmen 30 keine andere Möglichkeit gibt, als eine im Wesentlichen vollständig gestreckte Form einzunehmen. Wird die Handhabungseinrichtung in den Körper 20 hinein in Richtung auf das zu entfernende Organ 22 (Fig. 1) und somit in Richtung ihrer proximalen Endstellung bewegt, so bewirkt die Formgebung des ersten nach außen gekrümmten Abschnittes 30c zunächst ein Aufspreizen der Greifarme 30 im Bereich ihres proximalen Endes 30a. Die Spreizbewegung hält so lange an, bis der mittlere Abschnitt 30e der Greifarme 30 mit seiner radial am weitesten außen liegenden Stelle aus der Hülse 8 austritt, sodass zu diesem Zeitpunkt die Greifarme 30 eine ähnliche gespreizte Stellung einnehmen, wie in Fig. 11 gezeigt ist. Bei fortgesetzter Bewegung aus der Hülse 8 heraus bewirkt die Formgebung der Greifarme 30 zwischen dem mittleren Abschnitt 30e und dem zweiten gekrümmten Abschnitt 30d in Richtung des distalen Endes 30b wieder ein Schließen der Greifarme 30 an ihren proximalen Enden 30a, und zwar spätestens, wenn der bogenförmige mittlere Abschnitt 30e der Greifarme 30 außerhalb der Hülse 8 freiliegt und somit die Greifarme 30 keinerlei von der Hülse 8 verursachten Zwangseinflüssen mehr unterliegen; dann nehmen die Greifarme 30 etwa eine Stellung ein, wie sie in Fig. 12 gezeigt ist.

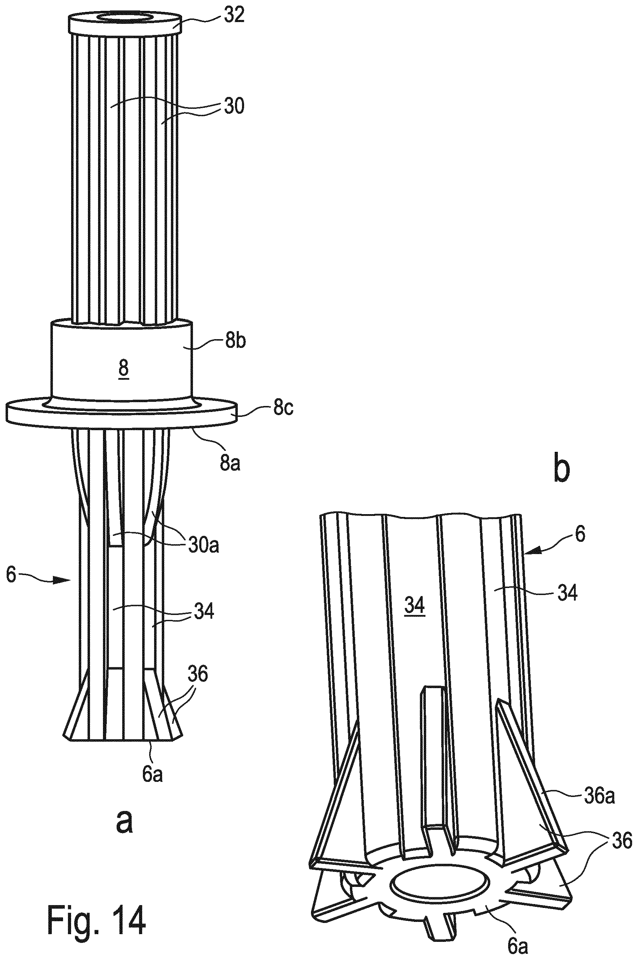

In Fig. 14 ist schematisch in perspektivischer Ansicht ein Teil einer Handhabungseinrichtung mit Greifarmen 30 als Teil einer Vorrichtung zur Entfernung von Organen aus dem menschlichen oder tierischen Körper gemäß einer weiteren bevorzugten Ausführung abgebildet. Wie Fig. 14a erkennen lässt, unterscheidet sich die dort dargestellte Ausführung von der Ausführung gemäß Fig. 12 dadurch, dass im Bereich des proximalen Endes 6a des inneren Rohres 6 in jeder Führungsnut 34 ein sogenanntes Spreizelement 36 angeordnet ist. Die Spreizelemente 36 haben die Form eines Dreiecks und weisen an ihrer Außenseite eine Führungsfläche 36a auf, die vom Boden der zugehörigen Führungsnut 34 in Richtung auf das proximale Ende 6a des inneren Rohres 6 ansteigt, sodass der radiale Abstand der Führungsfläche 36a der Spreizelemente 36 von der Mittelachse des inneren Rohres 6 am proximalen Ende 6a des inneren Rohres 6 größer als der Radius des inneren Rohres 6 ist. Bei Bewegung der Greifarme 30 aus der Hülse 8 hinaus und dabei entlang der Führungsnuten 34 gelangen die Greifarme 30 zunächst mit ihrem proximalen Ende 30a in Anlage an die Führungsflächen 36a der Spreizelemente 36 und werden bei fortgesetzter Bewegung entlang der Führungsfläche 36a der Spreizelemente 36 nach außen gedrückt, sodass die Spreizelemente 36 wie ein Keil oder Konus zur Aufspreizung der daran dann anliegenden Greifarme 30 wirken.

Wie Fig. 14 ferner erkennen lässt, sind die Spreizelemente 36 in einem Abstand voneinander entsprechend dem Abstand der Greifarme 30 angeordnet und ist jedem Greifarm 30 ein Spreizelement zugeordnet. Zum Lösen der Greifarme 30 von den Spreizelementen 36 sind das innere Rohr 6 einerseits und/oder die Greifarme 30 andererseits mit einer Relativbewegung zueinander beaufschlagbar, um die bis dahin auf den Spreizelementen 36 aufliegenden Greifarme 30 in eine Position seitlich neben den Spreizelementen 36 zu verbringen. Somit sorgt ein seitliches Verschieben des inneren Rohres 6 gegenüber den Greifarmen 30 oder ein seitliches Verschieben der Greifarme 30 durch Beaufschlagung des die Greifarme 30 an ihrem distalen Ende 30b miteinander verbindenden Ringes 32 mit einer Drehbewegung oder ein entsprechend gegenseitiges seitliches Verschieben des inneren Rohres 6 und der Greifarme 30 zueinander für ein Abrutschen der Greifarme 30 von den Spreizelementen und dadurch für ein Schließen der Greifarme 30. Wie insbesondere Fig. 14b im Einzelnen erkennen lässt, können die Spreizelemente 36 mit einer geringeren Breite als die Führungsnuten 34 versehen und innerhalb der Führungsnuten 34 seitlich angeordnet sein, sodass bei einem Abrutschen der Greifarme 30 von den Spreizelementen 36 die Greifarme 30 wieder von den Führungsnuten 34 aufgenommen werden können; hierzu ist es allerdings erforderlich, dass die Breite der Greifarme 30 nicht größer als die verbliebene lichte Breite der Führungsnuten 34 im Bereich der Spreizelemente 36 ist.

## Patentansprüche

1. Vorrichtung zur Entfernung von Organen aus dem menschlichen oder tierischen Körper,
mit einem Rohr (6), das ein proximales Ende (6a) und ein distales Ende (6b) aufweist und zum teilweisen Einführen mit seinem proximalen Ende (6a) in den Körper (20) vorgesehen ist,
mit einer das Rohr (6) zumindest abschnittsweise umschließenden Schlauchhülle (12), die ein proximales Ende (12a) und ein distales Ende (12b), mit dem sie am Rohr (6) befestigt ist, aufweist,
mit einer Handhabungseinrichtung (30), die am Rohr (6) angeordnet und ausgebildet ist, die Schlauchhülle (12) an ihrem proximalen Ende (12a) zu öffnen oder aufzuweiten und um das Organ (22) herum zu führen oder zu legen,
mit einer Schließeinrichtung (26, 28), die zum Verschließen des proximalen Endes (12a) der Schlauchhülle (12) ausgebildet ist, und
mit einer Zerkleinerungseinrichtung (14, 16), die innerhalb des Rohres (6), vorzugsweise im Bereich seines proximalen Endes (6a), vorgesehen ist, **dadurch gekennzeichnet, dass** das distale Ende (6b) des Rohres (6) an eine Saugluftquelle anschließbar und das proximale Ende (6a) des Rohres (6) für einen Saugeingriff mit dem zu entfernenden Organ (22) ausgebildet ist.

2. Vorrichtung nach Anspruch 1, bei welcher die Handhabungseinrichtung (30) gegenüber dem Rohr (6) in dessen Längsrichtung zwischen einer ausgefahrenen proximalen Endstellung und einer zurückgezogenen distalen Endstellung, bevorzugt gegenüber dem Rohr (6) in dessen Querrichtung, bewegbar angeordnet ist.

3. Vorrichtung nach Anspruch 1 oder 2, bei welcher die Handhabungseinrichtung einander gegenüberliegende Greifarme (30) aufweist, die jeweils ein proximales freies Ende (30a) und ein distales Ende (30b) aufweisen und zum lösbaren Greifen der Schlauchhülle (12), bevorzugt im Bereich ihres proximalen Endes (12a), ausgebildet sind, wobei vorzugsweise die Greifarme, bevorzugt im Bereich deren proximalen Endes, Fixiermittel aufweisen, die ausgebildet sind, die Schlauchhülle, bevorzugt mit ihrem proximalen Ende, an den Greifarmen lösbar zu fixieren, und/oder wobei insbesondere die Greifarme (30) in Längsrichtung des Rohres (6) orientiert sind.

4. Vorrichtung nach Anspruch 3, bei welcher die Greifarme (30) winklig, bevorzugt etwa quer, zur Längserstreckung des Rohres (6) elastisch sind, wobei vorzugsweise in einem entspannten Zustand der Greifarme (30) die proximalen Enden (30a) von jeweils einander gegenüberliegenden Greifarmen (30) einen Abstand voneinander aufweisen, der kleiner als der Durchmesser des Rohres (6) ist, und ein zwischen den proximalen und distalen Enden (30a, 30b) befindlicher mittlerer Abschnitt (30e) der Greifarme (30) in Bezug auf das Rohr (6) nach außen gekrümmt ist, so dass der Abstand der mittleren Abschnitte (30e) von jeweils einander gegenüberliegenden Greifarmen (30) größer als der Abstand zwischen den proximalen Enden (30a) dieser Greifarme (30) ist, welcher bevorzugt zwischen den mittleren Abschnitten (30e) von jeweils einander gegenüberliegenden Greifarmen (30) größer als der Durchmesser des Rohres (6) ist.

5. Vorrichtung nach Anspruch 3 oder 4, bei welcher die Greifarme (30) länger als das Rohr (6) ausgebildet sind.

6. Vorrichtung nach Anspruch 2 sowie mindestens einem der Ansprüche 3 bis 5, bei welcher die Außenseite des Rohres (6) mit in dessen Längsrichtung verlaufenden Führungsnuten (34) versehen ist, in denen die Greifarme (30) in deren Längsrichtung beweglich aufgenommen sind, und die Anordnung so getroffen ist, dass in der proximalen Endstellung der Handhabungseinrichtung die Greifarme (30) mit einem sich an ihr proximales Ende (30a) anschließenden Abschnitt über das proximale Ende (6a) des Rohres (6) hinausragen und somit freiliegen.

7. Vorrichtung nach Anspruch 2 sowie nach mindestens einem der Ansprüche 3 bis 6, bei welcher die Handhabungseinrichtung gegenüber dem Rohr (6) in dessen Querrichtung bewegbar angeordnet ist und Spreizelemente (36) aufweist, die ausgebildet sind, die Greifarme (30) während der Bewegung der Handhabungseinrichtung in ihre proximale Endstellung zumindest zeitweise voneinander weg zu spreizen.

8. Vorrichtung nach Anspruch 7, bei welcher die Spreizelemente (36) am Rohr (6) im Bereich dessen proximalen Endes (6a) angeordnet sind und eine von der Außenseite des Rohres (6) zu dessen proximalen Ende (6a) hin ansteigende Führungsfläche (36a) aufweisen, an die die Greifarme (30) in berührende Anlage bringbar sind.

9. Vorrichtung nach den Ansprüchen 6 und 8, bei welcher die Spreizelemente (36) in den Führungsnuten (34) angeordnet sind und deren Führungsfläche (36a) vom Boden der Führungsnuten (34) ansteigen.

10. Vorrichtung nach Anspruch 8 oder 9, bei welcher die Spreizelemente (36) in einem Abstand voneinander angeordnet sind, jedem Greifarm (30) ein Spreizelement (36) zugeordnet ist und zum Lösen der Greifarme (30) von den Spreizelementen (36) das Rohr (6) einerseits und/oder die Greifarme (30) andererseits mit einer Relativbewegung zueinander beaufschlagbar sind, um die Greifarme (30) in eine Position seitlich neben den Spreizelementen (36) zu verbringen.

11. Vorrichtung nach mindestens einem der Ansprüche 3 bis 10, bei welcher die Greifarme (30) im Bereich ihres distalen Endes (30b) miteinander verbunden sind, wobei bevorzugt ein ringförmiges Element (32) vorgesehen ist, an dem die Greifarme (30) mit ihrem distalen Ende (30b) befestigt sind.

12. Vorrichtung nach mindestens einem der vorangegangenen Ansprüche, bei welcher die Schließeinrichtung an der Handhabungseinrichtung vorgesehen ist.

13. Vorrichtung nach Ansprüchen 3 und 12, bei welcher die Schließeinrichtung Schließmittel aufweist, die an den Greifarmen, bevorzugt an deren freien oder proximalen Enden, vorgesehen sind.

14. Vorrichtung nach mindestens einem der vorangegangenen Ansprüche, bei welcher die Schließeinrichtung am proximalen Ende (12a) der Schlauchhülle (12) vorgesehene Augen oder Ösen (26) und mindestens einen durch die Augen oder Ösen (26) ziehbaren Faden oder Draht (28) aufweist und/oder, bei welcher die Zerkleinerungseinrichtung mindestens ein rotierbar gelagertes Schneidmesser (14) aufweist.

15. Vorrichtung nach mindestens einem der vorangegangenen Ansprüche, ferner mit einer Hülse (8) zur Anordnung auf der Oberfläche (18) des Körpers (20) oder zum Einsetzen in die Oberfläche (18) des Körpers (20), wobei das Rohr (6) sich durch die Hülse (8) erstreckt und gegenüber der Hülse (8) bewegbar ist.

16. Vorrichtung nach den Ansprüchen 3 und 15, bei welcher die Greifarme (30) zwischen der Innenseite der Hülse (8) und der Außenseite des Rohres (6) angeordnet sind und sich in berührender Anlage an der Innenseite der Hülse (8) und der Außenseite des Rohres (6) befinden.

17. Vorrichtung nach den Ansprüchen 2 und 3 sowie nach Anspruch 15 oder 16, bei welcher die Innenseite der Hülse mit in Bewegungsrichtung der Handhabungseinrichtung verlaufenden Führungsnuten versehen ist, in denen die Greifarme in deren Längsrichtung beweglich angeordnet sind, und die Anordnung so getroffen ist, dass in der proximalen Endstellung der Handhabungseinrichtung die Greifarme mit einem an ihr proximales Ende anschließenden Abschnitt über das proximale Ende des Rohres hinausragen und somit freiliegen.

18. Vorrichtung nach mindestens einem der Ansprüche 15 bis 17, bei welcher die Hülse (8) einen flanschartigen Rand (8c) zur Auflage auf die Oberfläche (18) des Körpers (20) aufweist.

19. Vorrichtung nach mindestens einem der vorangegangenen Ansprüche, bei welcher die Schlauchhülle (12) als Doppelhülle mit einer Innenhülle (12c) und einer die Innenhülle (12c) in einem Abstand unter Bildung eines Zwischenraumes (12e) umgebenden Außenhülle (12d) ausgebildet ist und an den zwischen Innenhülle (12c) und Außenhülle (12d) gebildeten Zwischenraum (12e) eine Druckluftquelle anschließbar ist, wobei bevorzugt die Schließeinrichtung zum Verschließen des proximalen Endes der Innenhülle und zum Verschließen des proximalen Endes der Außenhülle (12d) oder zum gemeinsamen Verschließen der proximalen Enden der Innenhülle und der Außenhülle (12d) ausgebildet ist.

20. Vorrichtung nach Anspruch 19, bei welcher die Handhabungseinrichtung (30) zumindest abschnittsweise in dem zwischen Innenhülle (12c) und Außenhülle (12d) gebildeten Zwischenraum (12e) angeordnet ist.

21. Vorrichtung nach Anspruch 19 oder 20, bei welcher am proximalen Ende (6a) der Schlauchhülle (12) die Innenhülle (12c) mit der Außenhülle (12d) dichtend verbunden ist.

22. Vorrichtung nach mindestens einem der vorangegangenen Ansprüche, bei welcher das Rohr (6) als Innenrohr ausgebildet ist, das von einem Außenrohr in einem Abstand unter Bildung eines Zwischenraumes (12e) umgeben ist,
das Innenrohr gegenüber dem Außenrohr bewegbar und dabei mit einem zu seinem proximalen Ende (12a) benachbarten Abschnitt aus dem proximalen Ende (12a) des Außenrohres ausziehbar angeordnet ist,
die Schlauchhülle (12) vor Benutzung im Wesentlichen in dem Zwischenraum (12e) zwischen Innenrohr und Außenrohr angeordnet und durch Ausziehen des Innenrohres (6) aus dem Außenrohr freilegbar ist, und
die Zerkleinerungseinrichtung (14, 16) innerhalb des Innenrohres (6) im Bereich seines proximalen Endes (6a) vorgesehen ist.

23. Vorrichtung den Ansprüchen 15 und 22, bei welcher die Hülse (8) am proximalen Ende (6a) des Außenrohres angeordnet oder vom Außenrohr gebildet ist.

24. Vorrichtung nach Anspruch 22 oder 23, bei welcher die Handhabungseinrichtung (30) an der Außenseite des Innenrohrs angeordnet und ausgebildet ist.

25. Vorrichtung nach Anspruch 22 oder 23, bei welcher die Handhabungseinrichtung (30) am Außenrohr angeordnet und ausgebildet ist.

26. Vorrichtung nach einem der Ansprüche 22 bis 24, bei welcher die Schlauchhülle (12) mit ihrem distalen Ende (12b) am Innenrohr befestigt ist oder das Innenrohr so ausgebildet ist, dass es beim Ausziehen aus dem Außenrohr die Schlauchhülle (12) mitnimmt und dadurch im Wesentlichen freilegt.

27. Vorrichtung nach Anspruch 19 sowie nach einem der Ansprüche 22 bis 24, bei welcher die Innenhülle (12c) am Innenrohr und die Außenhülle (12d) am Außenrohr befestigt ist und die Druckluftquelle an den zwischen Innenrohr und Außenrohr gebildeten Zwischenraum (12e) anschließbar ist.

## Claims

1. Device for removing organs from the human or animal body,
comprising a tube (6) which has a proximal end (6a) and a distal end (6b) and is intended to be partially inserted into the body (20) with its proximal end (6a),
with a tubular casing (12), which surrounds the tube (6) at least in sections, and which has a proximal end (12a) and a distal end (12b), by which it is attached to the tube (6),
with a handling device (30), which is arranged on the tube (6) and is designed to open or widen the tubular casing (12) at its proximal end (12a) and to guide or place it around the organ (22),
with a closing device (26, 28), which is designed to close the proximal end (12a) of the tubular casing (12), and
with a comminuting device (14, 16) which is provided inside the tube (6), preferably in the area of its proximal end (6a),
**characterised in that** the distal end (6b) of the tube (6) can be connected to a suction air source and the proximal end (6a) of the tube (6) is designed for engaging by suction with the organ (22) to be removed.

2. Device according to claim 1, wherein the handling device (30) is arranged to be movable relative to the tube (6) in its longitudinal direction between an extended proximal end position and a retracted distal end position, preferably relative to the tube (6) in its transverse direction.

3. Device according to claim 1 or 2, wherein the handling device has opposite gripping arms (30), which each have a proximal free end (30a) and a distal end (30b), and are designed for releasably gripping the tubular casing (12), preferably in the region of its proximal end (12a), wherein advantageously the gripping arms, preferably in the region of their proximal end, have fixing means, which are designed to fix the tubular casing in a releasable manner on the gripping arms, preferably at its proximal end, and/or wherein in particular the gripping arms (30) are oriented in longitudinal direction of the tube (6).

4. Device according to claim 3, wherein the gripping arms (30) are elastic at an angle, preferably approximately perpendicular, to the longitudinal extension of the tube (6), wherein preferably in a relaxed state of the gripping arms (30) the proximal ends (30a) of respective opposing gripping arms (30) are spaced apart from one another by a distance which is smaller than the diameter of the tube (6), and a central section (30e) of the gripping arms (30) that is located between the proximal and the distal ends (30a, 30b) is curved outwards with respect to the tube (6), so that the distance between the central sections (30e) of respectively opposing gripping arms (30) is greater than the distance between the proximal ends (30a) of said gripping arms (30), which preferably between the central sections (30e) of respective opposing gripping arms (30) is greater than the diameter of the tube (6).

5. Device according to claim 3 or 4, wherein the gripping arms (30) are designed to be longer than the tube (6).

6. Device according to claim 2 and at least one of claims 3 to 5, wherein the outer surface of the tube (6) is provided with guide grooves (34) extending in its longitudinal direction, in which the gripping arms (30) are accommodated moveably in longitudinal direction, and the arrangement is such that in the proximal end position of the handling device, the gripping arms (30) with a section adjoining their proximal end (30a) protrude beyond the proximal end (6a) of the tube (6) and consequently are exposed.

7. Device according to claim 2 and according to at least one of claims 3 to 6, wherein the handling device is arranged to be movable relative to the tube (6) in its transverse direction and comprises spreading elements (36), which are designed to spread the gripping arms (30) apart from one another at least temporarily during the movement of the handling device into its proximal end position.

8. Device according to claim 7, wherein the spreading elements (36) are arranged on the tube (6) in the region of its proximal end (6a), and have a guide surface (36a) which rises from the outside of the tube (6) to its proximal end (6a), to which the gripping arms (30) can be brought into contact.

9. Device according to claims 6 and 8, wherein the spreading elements (36) are arranged in the guide grooves (34) and the guide surfaces (36a) of which rise from the bottom of the guide grooves (34).

10. Device according to claim 8 or 9, wherein the spreading elements (36) are arranged at a distance from one other, a spreading element (36) is assigned to each gripping arm (30) and for releasing the gripping arms (30) from the spreading elements (36) the tube (6) on the one hand and/or the gripping arms (30) on the other hand can be made to impinge on one other in a relative movement, in order to position the gripping arms (30) in a lateral position next to the spreading elements (36).

11. Device according to at least one of claims 3 to 10, wherein the gripping arms (30) are connected to each other in the region of their distal end (30b), wherein preferably an annular element (32) is provided, to which the gripping arms (30) are attached at their distal end (30b).

12. Device according to at least one of the preceding claims, wherein the closing device is provided on the handling device.

13. Device according to claims 3 and 12, wherein the closing device has closing means, which are provided on the gripping arms, preferably on their free or proximal ends.

14. Device according to at least one of the preceding claims, wherein the closing device has eyelets or loops (26) at the proximal end (12a) of the tubular casing (12) and has at least one thread or wire which can be pulled through the eyelets or loops (26), and/or wherein the comminuting device has least one rotatably mounted cutting blade (14).

15. Device according to at least one of the preceding claims, also with a sleeve (8) for arranging on the surface (18) of the body (20) or for inserting into the surface (18) of the body (20), wherein the tube (6) extends through the sleeve (8) and can move relative to the sleeve (8).

16. Device according to claims 3 and 15, wherein the gripping arms (30) are arranged between the inside of the sleeve (8) and the outside of the tube (6) and are in contact with the inside of the sleeve (8) and the outside of the tube (6).

17. Device according to claims 2 and 3 and according to claim 15 or 16, wherein the inside of the sleeve is provided with guiding grooves running in the direction of movement of the handling device, in which the gripping arms are arranged to be movable in their longitudinal direction, and the arrangement is such that in the proximal end position of the handling device the gripping arms project by a section adjoining their proximal end over the proximal end of the tube and are thus exposed.

18. Device according to at least one of claims 15 to 17, wherein the sleeve (8) has a flange-like edge (8c) for bearing on the surface (18) of the body (20).

19. Device according to at least one of the preceding claims, wherein the tubular casing (12) is designed as a double casing with an inner casing (12c) and an outer casing (12d) surrounding the inner casing (12c) at a distance forming an intermediate space (12e) and a source of compressed air can be connected to the intermediate space (12e) formed between the inner casing (12c) and outer casing (12d), wherein preferably the closing device is designed for closing the proximal end of the inner casing and for closing the proximal end of the outer casing (12d) or for jointly closing the proximal ends of the inner casing and the outer casing (12d).

20. Device according to claim 19, wherein the handling device (30) is arranged at least partly in the intermediate space (12e) formed between the inner casing (12c) and outer casing (12d).

21. Device according to claim 19 or 20, wherein at the proximal end (6a) of the tubular casing (12) the inner casing (12c) is connected in a sealing manner to the outer casing (12d).

22. Device according to at least one of the preceding claims, wherein the tube (6) is designed as an inner tube, which is surrounded by an outer tube at a distance forming an intermediate space (12e),
the inner tube is arranged to be movable relative to the outer tube and thereby with a section adjacent to its proximal end (12a) is arranged to be removable from the proximal end (12a) of the outer tube,
the tubular casing (12) is arranged before use substantially in the intermediate space (12e) between the inner tube and outer tube and can be exposed by removing the inner tube (6) from the outer tube, and the comminuting device (14, 16) is provided inside the inner tube (6) in the region of its proximal end (6a).

23. Device according to claims 15 and 22, wherein the sleeve (8) is arranged on the proximal end (6a) of the outer tube or is formed by the outer tube.

24. Device according to claim 22 or 23, wherein the handling device (30) is arranged and formed on the outside of the inner tube.

25. Device according to claim 22 or 23, wherein the handling device (30) is arranged and formed on the outer tube.

26. Device according to any of claims 22 to 24, wherein the tubular casing (12) is secured at its distal end (12b) to the inner tube or the inner tube is designed so that when removed from the outer tube it takes the tubular casing (12) with it and it is thereby substantially uncovered.

27. Device according to claim 19 and according to any of claims 22 to 24, wherein the inner casing (12c) is secured onto the inner tube and the outer casing (12d) is secured onto the outer tube and the source of compressed air can be connected to the intermediate space (12e) formed between the inner tube and outer tube.

## Revendications

1. Dispositif pour enlever des organes du corps humain ou animal,
avec un tube (6), qui présente une extrémité proximale (6a) et une extrémité distale (6b) et est prévu pour être introduit en partie par son extrémité proximale (6a) dans le corps (20),
avec une gaine tubulaire flexible (12) entourant au moins par endroits le tube (6), qui présente une extrémité proximale (12a) et une extrémité distale (12b), par laquelle elle est fixée au niveau du tube (6),
avec un système de manipulation (30), qui est disposé au niveau du tube (6) et est réalisé pour ouvrir ou élargir la gaine tubulaire flexible (12) au niveau de son extrémité proximale (12a) et la guider ou la placer tout autour de l'organe (22),
avec un système de fermeture (26, 28), qui est réalisé pour fermer l'extrémité proximale (12a) de la gaine tubulaire flexible (12), et
avec un système de broyage (14, 16), qui est prévu à l'intérieur du tube (6), de préférence dans la zone de son extrémité proximale (6a),
**caractérisé en ce que** l'extrémité distale (6b) du tube (6) peut être raccordée à une source d'air d'aspiration et l'extrémité proximale (6a) du tube (6) est réalisée pour une prise par aspiration avec l'organe (22) à enlever.

2. Dispositif selon la revendication 1, où le système de manipulation (30) est disposé de manière à pouvoir être déplacé par rapport au tube (6) dans son sens longitudinal entre une position finale proximale sortie et une position finale distale rentrée, de manière préférée par rapport au tube (6) dans son sens transversal.

3. Dispositif selon la revendication 1 ou 2, où le système de manipulation présente des bras de préhension (30) se faisant face l'un l'autre, qui présentent respectivement une extrémité libre proximale (30a) et une extrémité distale (30b) et qui sont réalisés pour saisir de manière amovible la gaine tubulaire flexible (12), de manière préférée dans la zone de son extrémité proximale (12a), dans lequel de préférence les bras de préhension présentent de manière préférée dans la zone de leur extrémité proximale des moyens de blocage, qui sont réalisés pour bloquer de manière amovible la gaine tubulaire flexible de manière préférée par son extrémité proximale au niveau des bras de préhension, et/ou dans lequel en particulier les bras de préhension (30) sont orientés dans le sens longitudinal du tube (6) .

4. Dispositif selon la revendication 3, où les bras de préhension (30) sont élastiques de manière angulaire, de manière préférée à peu près de manière transversale, par rapport à l'extension longitudinale du tube (6), dans lequel de préférence dans un état détendu des bras de préhension (30), les extrémités proximales (30a) de bras de préhension (30) se faisant face les uns les autres respectivement présentent une distance, les unes des autres, qui est inférieure au diamètre du tube (6), et une section centrale (30e), se trouvant entre les extrémités proximale et distale (30a, 30b), des bras de préhension (30) est incurvée vers l'extérieur par rapport au tube (6) de sorte que la distance des sections centrales (30e) de bras de préhension (30) se faisant face respectivement les uns les autres respectivement est supérieure à la distance entre les extrémités proximales (30a) desdits bras de préhension (30), laquelle est supérieure de manière préférée entre les sections centrales (30e) de bras de préhension (30) se faisant face les uns les autres respectivement au diamètre du tube (6).

5. Dispositif selon la revendication 3 ou 4, où les bras de préhension (30) sont réalisés de manière plus longue que le tube (6).

6. Dispositif selon la revendication 2 et selon au moins l'une quelconque des revendications 3 à 5, où le côté extérieur du tube (6) est pourvu de rainures de guidage (34) s'étendant dans son sens longitudinal, dans lesquelles les bras de préhension (30) sont logés de manière mobile dans leur sens longitudinal, et l'ensemble est tel que, dans la position finale proximale du système de manipulation, les bras de préhension (30) dépassent par une section se raccordant à leur extrémité proximale (30a) de l'extrémité proximale (6a) du tube (6) et sont ainsi dégagés.

7. Dispositif selon la revendication 2 et selon au moins l'une quelconque des revendications 3 à 6, où le système de manutention est disposé de manière à pouvoir être déplacé dans son sens transversal par rapport au tube (6) et présente des éléments d'écartement (36), qui sont réalisés pour éloigner les uns des autres par écartement au moins de manière temporaire les bras de préhension (30) pendant le déplacement du système de manipulation dans sa position finale proximale.

8. Dispositif selon la revendication 7, où les éléments d'écartement (36) sont disposés au niveau du tube (6) dans la zone de son extrémité proximale (6a) et présentent une surface de guidage (36a) croissante depuis le côté extérieur du tube (6) vers son extrémité proximale (6a), au niveau de laquelle les bras de préhension (30) peuvent être amenés en appui par contact.

9. Dispositif selon les revendications 6 et 8, où les éléments d'écartement (36) sont disposés dans les rainures de guidage (34) et leur surface de guidage (36a) est croissante depuis le fond des rainures de guidage (34).

10. Dispositif selon la revendication 8 ou 9, où les éléments d'écartement (36) sont disposés à une distance les uns des autres, un élément d'écartement (36) est associé à chaque bras de préhension (30) et le tube (6) d'une part et/ou les bras de préhension (30) d'autre part peuvent être soumis à une action par un déplacement relatif les uns par rapport aux autres pour détacher les bras de préhension (30) des éléments d'écartement (36) afin d'amener les bras de préhension (30) dans une position latéralement à côté des éléments d'écartement (36).

11. Dispositif selon au moins l'une quelconque des revendications 3 à 10, où les bras de préhension (30) sont reliés les uns aux autres dans la zone de leur extrémité distale (30b), dans lequel de manière préférée un élément annulaire (32) est prévu,
au niveau duquel les bras de préhension (30) sont fixés par leur extrémité distale (30b).

12. Dispositif selon au moins l'une quelconque des revendications précédentes, où le système de fermeture est prévu au niveau du système de manutention.

13. Dispositif selon les revendications 3 et 12, où le système de fermeture présente des moyens de fermeture, qui sont prévus au niveau des bras de préhension, de manière préférée au niveau de leurs extrémités libres ou proximales.

14. Dispositif selon au moins l'une quelconque des revendications précédentes, où le système de fermeture présente des œillets (26) prévus au niveau de l'extrémité proximale (12a) de la gaine tubulaire flexible (12) et au moins un fil ou fil métallique (28) pouvant être tiré à travers l'œillet (26) et/ou où le système de broyage présente au moins une lame tranchante (14) montée de manière rotative.

15. Dispositif selon au moins l'une quelconque des revendications précédentes, en outre avec une douille (8) destinée à être disposée sur la surface (18) du corps (20) ou destinée à être insérée dans la surface (18) du corps (20), dans lequel le tube (6) s'étend à travers la douille (8) et peut être déplacé par rapport à la douille (8).

16. Dispositif selon les revendications 3 et 15, où les bras de préhension (30) sont disposés entre le côté intérieur de la douille (8) et le côté extérieur du tube (6) et se trouvent en appui par contact au niveau du côté intérieur de la douille (8) et du côté extérieur du tube (6).

17. Dispositif selon les revendications 2 et 3 et ainsi que selon la revendication 15 ou 16, où le côté intérieur de la douille est pourvu de rainures de guidage s'étendant dans le sens de déplacement du système de manipulation, dans lesquelles les bras de préhension sont disposés de manière mobile dans leur sens longitudinal, et l'agencement est tel que, dans la position finale proximale du système de manipulation, les bras de préhension dépassent, par une section se raccordant à leur extrémité proximale, de l'extrémité proximale du tube et sont ainsi dégagés.

18. Dispositif selon au moins l'une quelconque des revendications 15 à 17, où la douille (8) présente un bord (8c) de type flasque destiné à être placé sur la surface (18) du corps (20).

19. Dispositif selon au moins l'une quelconque des revendications précédentes, où la gaine tubulaire flexible (12) est réalisée en tant que double gaine avec une gaine intérieure (12c) et une gaine extérieure (12d) entourant la gaine intérieure (12c) à une distance en formant un espace intermédiaire (12e) et une source d'air comprimé peut être raccordée à l'espace intermédiaire (12e) formé entre la gaine intérieure (12c) et la gaine extérieure (12d), dans lequel de manière préférée le système de fermeture est réalisé pour fermer l'extrémité proximale de la gaine intérieure et pour fermer l'extrémité proximale de la gaine extérieure (12d) ou pour fermer conjointement les extrémités proximales de la gaine intérieure et de la gaine extérieure (12d).

20. Dispositif selon la revendication 19, où le système de manipulation (30) est disposé au moins par endroits dans l'espace intermédiaire (12e) formé entre la gaine intérieure (12c) et la gaine extérieure (12d).

21. Dispositif selon la revendication 19 ou 20, où la gaine intérieure (12c) est reliée de manière étanche à la gaine extérieure (12d) au niveau de l'extrémité proximale (6a) de la gaine tubulaire flexible (12).

22. Dispositif selon au moins l'une quelconque des revendications précédentes, où le tube (6) est réalisé en tant que tube intérieur, qui est entouré d'un tube extérieur à une distance en formant un espace intermédiaire (12e),
le tube intérieur peut être déplacé par rapport au tube extérieur et est disposé ce faisant de manière à pouvoir être retiré de l'extrémité proximale (12a) du tube extérieur par une section adjacente par rapport à son extrémité proximale (12a),
la gaine tubulaire flexible (12) est disposée avant l'utilisation sensiblement dans l'espace intermédiaire (12e) entre le tube intérieur et le tube extérieur et peut être dégagée en retirant le tube intérieur (6) hors du tube extérieur, et
le système de broyage (14, 16) est prévu à l'intérieur du tube intérieur (6) dans la zone de son extrémité proximale (6a).

23. Dispositif selon les revendications 15 et 22, où la douille (8) est disposée au niveau de l'extrémité proximale (6a) du tube extérieur ou est formée par le tube extérieur.

24. Dispositif selon la revendication 22 ou 23, où le système de manipulation (30) est disposé et réalisé au niveau du côté extérieur du tube intérieur.

25. Dispositif selon la revendication 22 ou 23, où le système de manipulation (30) est disposé et réalisé au niveau du tube extérieur.

26. Dispositif selon l'une quelconque des revendications 22 à 24, où la gaine tubulaire flexible (12) est fixée au niveau du tube intérieur par son extrémité distale (12b) ou le tube intérieur est réalisé de telle sorte que, lors du retrait hors du tube extérieur, il entraîne la gaine tubulaire flexible (12) et la dégage ainsi sensiblement.

27. Dispositif selon la revendication 19 et selon l'une quelconque des revendications 22 à 24, où la gaine intérieure (12c) est fixée au niveau du tube intérieur et la gaine extérieure (12d) est fixée au niveau du tube extérieur et la source d'air comprimé peut être raccordée à l'espace intermédiaire (12e) formé entre le tube intérieur et le tube extérieur.
